(19) Europäisches Patentamt
European Patent Office
Office européen des brevets

(11) **EP 3 245 992 B1**

(12) **EUROPEAN PATENT SPECIFICATION**

(45) Date of publication and mention
of the grant of the patent:
**04.12.2019 Bulletin 2019/49**

(51) Int Cl.:
*A61F 13/49* (2006.01)    *A61F 13/15* (2006.01)
*A61F 13/53* (2006.01)    *B32B 27/00* (2006.01)
*B32B 27/12* (2006.01)    *B32B 27/20* (2006.01)

(21) Application number: **16737316.6**

(22) Date of filing: **12.01.2016**

(86) International application number:
**PCT/JP2016/050625**

(87) International publication number:
**WO 2016/114245 (21.07.2016 Gazette 2016/29)**

(54) **ABSORBENT ARTICLE**

ABSORBIERENDER GEGENSTAND

ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2015 JP 2015004631**

(43) Date of publication of application:
**22.11.2017 Bulletin 2017/47**

(73) Proprietors:
• **SDP Global Co., Ltd.**
**Tokyo 103-0023 (JP)**
• **Toyobo Co., Ltd.**
**Osaka-shi**
**Osaka 530-8230 (JP)**

(72) Inventors:
• **NISHIDA, Mitsuo**
**Osaka-shi**
**Osaka 530-8230 (JP)**
• **ISHIDA, Hidenobu**
**Tokyo 103-0023 (JP)**

(74) Representative: **Vossius & Partner**
**Patentanwälte Rechtsanwälte mbB**
**Siebertstrasse 3**
**81675 München (DE)**

(56) References cited:
**JP-A- H0 959 872       JP-A- H05 311 508**
**JP-A- H05 311 508     JP-A- H07 150 470**
**JP-A- H07 150 470     JP-A- H08 260 354**
**JP-A- 2011 156 384    US-A- 5 436 275**
**US-A1- 2011 301 560**

**Description**

TECHNICAL FIELD

The present invention relates to absorbent articles. More particularly, the present invention relates to absorbent articles to be used for a disposable diaper for children, a disposable diaper for adults, a sanitary napkin, a blood-retention agent for medical use, a pet sheet, a panty liner, an incontinence pad, a sweat absorbent sheet, a blood absorbent article for medical use, a wound-healing material, a wound-treating agent, a surgical drainage disposal agent, etc.

BACKGROUND ART

Absorbent articles have widely been known that have an aqueous-liquid absorbing part in which crosslinked polymer particles such as highly water-absorbent resin particles are mixed with hydrophilic fibers such as pulp and that have a liquid diffusion part, such as a tissue paper and a nonwoven fabric, disposed on an upper surface of the aqueous-liquid absorbing part (see, for example, Patent Document 1). Although an absorbent article with such a structure has excellent absorption performance, it is problematic in that when the attachment site moves or a certain force is continuously or discontinuously applied to the absorbing site during practical use, the absorbing part is ruptured or twisted, and as a result, the absorption performance is reduced and liquid leakage or rash of the skin, etc. derived therefrom are caused.

In order to suppress decrease in absorption performance caused by the rupture or twist of an absorbing part, there is a technique to increase fixability or tanglement of highly water-absorbent resin particles with pulp by optimizing the composition of a hot-melt adhesive used for fixation of the absorbing site (see, for example, Patent Document 2). However, these methods are not sufficient in retention of the shape of an absorber and do not have sufficient effects to suppress the decrease in absorption performance due to the rupture or twist of the absorbing part.

Patent Document 3 relates to an absorbent resin particle comprising a crosslinked polymer (A1) including, as an essential constituent unit, a water-soluble vinyl monomer (a1) and/or a hydrolyzable vinyl monomer (a2) and a crosslinking agent (b), wherein, in a measurement method of swelled volume per 1 g of the absorbent resin particle against physiological saline, a ratio (t2/t1) of the time (t2) until swelled volume reaches 40 ml to the time (t1) until the swelled volume reaches 5 ml is 5 to 20.

Patent Document 4 relates to a specific acrylonitrile polymer containing ≥95wt.% and <98wt.% of acrylonitrile unit which is spun by wet-spinning, the undried drawn fiber is subjected to wet heat-treatment at 120-150 °C and the product is crosslinked with a crosslinking monomer having functional group other than nitrile group to obtain a porous acrylonitrile fiber containing mutually interconnected fine pores having an average pore diameter of 1000-6000 Å and opened on the fiber surface, having a reduction ratio of the average pore diameter of ≤10% after the dry-heat treatment at 180 °C for 2hr.

Patent Document 5 relates to a specific undried yarn, obtained by wet spinning, of an acrylonitrile-based polymer having ≥95wt.% acrylonitrile which is subjected to wet heat treatment at 120-150 °C to give porous acrylonitrile-based yarn wherein pores with a specific average pore diameter are connected and open on the surface of fibers.

Patent Document 6 relates to a porous acrylonitrile polymer fiber which has a porous structure obtained by wet-spinning of an acrylonitrile polymer containing, in a chemically bonded manner, acrylonitrile of not lower than 95% by weight to obtain a stretched but undried fiber, which is then subjected to a wet-heat treatment at a temperature between 120 and 150°C and thereafter subjected to a crosslinking treatment, the micropores in the porous structure having an average pore diameter of 100 to 6000Å, said micropores being mutually connected and communicating with the fiber surface, and the degree of reduction of the average pore diameter after a dry heat treatment at 180 °C for 2 hours being not larger than 10%.

PRIOR ART DOCUMENTS

PATENT DOCUMENTS

Patent Document 1: Japanese Patent No. 3916852
Patent Document 2: Japanese Patent No. 5404959
Patent Document 3: US 2011/301560 A1
Patent Document 4: JP H07-150470 A
Patent Document 5: JP H05-311508 A
Patent Document 6: US 5,436,275 A

SUMMARY OF THE INVENTION

PROBLEM TO BE SOLVED BY THE INVENTION

An object of the present invention is to provide an absorbent article having excellent retention of the shape of an absorber even when an external force is applied thereto.

SOLUTIONS TO THE PROBLEM

The present inventors have studied in order to attain the object described above and, as a result, have achieved the present invention. That is, the present invention is an absorbent article comprising an aqueous-liquid absorbing part that contains crosslinked polymer particles (A) comprising, as essential constitutional units, a water-soluble vinyl monomer (a1) and/or a hydrolyzable vinyl monomer (a2), and a crosslinking agent (a3); and a nonwoven fabric (B) comprising, as an essential constitutional unit, porous fibers (b1), wherein the porous fibers (b1) are formed of an acrylonitrile-based polymer.

ADVANTAGES OF THE INVENTION

Because of the above-described structure, especially, because of the use of a nonwoven fabric (B) comprising, as an essential constituent, porous fibers (b1) as defined in the claims, the absorbent article of the present invention is superior to conventional absorbent articles in retention of the shape of an absorber even when an external force is applied thereto. Even if a certain force is continuously or discontinuously applied to an absorbing site, neither rupture nor twist of the absorbing part occurs and liquid leakage is not caused by deterioration of absorption performance, and rash of the skin, etc. derived therefrom are not caused.

MODE FOR CARRYING OUT THE INVENTION

The absorbent article of the present invention comprises an aqueous-liquid absorbing part that contains crosslinked polymer particles (A) comprising, as essential constitutional units, a water-soluble vinyl monomer (a1) and/or a vinyl monomer (a2) which turns into a water-soluble vinyl monomer (a1) through hydrolysis, and a crosslinking agent (a3); and a nonwoven fabric (B) comprising, as an essential constituent, porous fibers (b1) as defined in the claims.

In the present invention, the aqueous-liquid absorbing part is a part that performs absorption of an aqueous liquid, and examples of the aqueous liquid include body fluids, such as urine, sweat, and blood, and aqueous liquids to be used for various applications (e.g., industrial applications, medical applications, and agriculture, forestry and fisheries applications).

The crosslinked polymer (A) included in the aqueous-liquid absorbing part comprises, as essential constitutional units, a water-soluble vinyl monomer (a1) and/or a hydrolyzable vinyl monomer (a2), and a crosslinking agent (a3).

The water-soluble vinyl monomer (a1) is not particularly restricted and conventional vinyl monomers (e.g., Japanese Patent No. 3648553, JP-A-2003-165883, JP-A-2005-75982, and JP-A-2005-95759), etc. can be used.

As the vinyl monomer (a2) that turns into a water-soluble vinyl monomer (a1) through hydrolysis, conventional vinyl monomers (e.g., Japanese Patent No. 3648553, JP-A-2003-165883, JP-A-2005-75982, and JP-A-2005-95759), etc. can be used. The water-soluble vinyl monomer as used herein means a vinyl monomer having a property that at least 100 g of the monomer can be dissolved in 100 g of water at 25°C. Hydrolyzability means a property to be hydrolyzed by the action of water at 50°C and, according to need, of a catalyst (e.g., an acid or a base), thereby becoming water-soluble. Although the hydrolysis of the hydrolyzable vinyl monomer may be carried out during polymerization, after polymerization, or both during and after polymerization, after polymerization is preferred from the viewpoint of the molecular weight of absorbent resin particles to be obtained, etc.

Among these, water-soluble vinyl monomers (a1) are preferred from the viewpoint of absorption characteristics, etc., anionic vinyl monomers are more preferred, vinyl monomers having a carboxy (salt) group, a sulfo (salt) group, an amino group, a carbamoyl group, an ammonio group, or a mono-, di- or tri-alkylammonio group are still more preferred, vinyl monomers having a carboxy (salt) group or a carbamoyl group are even more preferred, (meth)acrylic acid (salt) and (meth)acrylamide are particularly preferred, (meth)acrylic acid (salt) is further particularly preferred, and acrylic acid (salt) is most preferred.

The "carboxy (salt) group" means a "carboxy group" or a "carboxylate group", and the "sulfo (salt) group" means a "sulfo group" or a "sulfonate group." The (meth)acrylic acid (salt) means acrylic acid, a salt of acrylic acid, methacrylic acid, or a salt of methacrylic acid, and the (meth)acrylamide means acrylamide or methacrylamide. Examples of such salts include salts of alkali metals (lithium, sodium, potassium, etc.), salts of alkaline earth metals (magnesium, calcium,

etc.), or ammonium ($NH_4$) salts. Among these salts, salts of alkali metals and ammonium salts are preferred from the viewpoint of absorption characteristics, etc., salts of alkali metals are more preferred, and sodium salts are particularly preferred.

When either one of a water-soluble vinyl monomer (a1) and the vinyl monomer (a2) is contained as a constitutional unit, a single species may be contained as a constitutional unit or, alternatively, two or more species may be contained as constitutional units, if necessary. The same also applies to the case where both a water-soluble vinyl monomer (a1) and the vinyl monomer (a2) are contained as constitutional units. When both the water-soluble vinyl monomer (a1) and the vinyl monomer (a2) are contained as constitutional units, their contained molar ratio (a1/a2) is preferably from 75/25 to 99/1, more preferably from 85/15 to 95/5, particularly preferably from 90/10 to 93/7, and most preferably from 91/9 to 92/8. Within such ranges, the absorption performance is further improved.

In addition to the water-soluble vinyl monomer (a1) and the vinyl monomer (a2), an additional vinyl monomer (a') copolymerizable with them can be contained as a constitutional unit of the crosslinked polymer particles (A).

The additional copolymerizable vinyl monomer (a') is not particularly restricted and conventional hydrophobic vinyl monomers (e.g., Japanese Patent No. 3648553, JP-A-2003-165883, JP-A-2005-75982, and JP-A-2005-95759), etc. can be used, and, for example, the following vinyl monomers (i) to (iii) can be used.

(i) Aromatic ethylenic monomers having 8 to 30 carbon atoms

Styrenes, such as styrene, alpha-methylstyrene, vinyltoluene, and hydroxystyrene, and halogenated forms of styrene, such as vinylnaphthalene and dichlorostyrene.

(ii) Aliphatic ethylene monomers having 2 to 20 carbon atoms

Alkenes [e.g., ethylene, propylene, butene, isobutylene, pentene, heptene, diisobutylene, octene, dodecene, and octadecene]; and alkadienes [e.g., butadiene and isoprene]; etc.

(iii) Alicyclic ethylene monomers having 5 to 15 carbon atoms

Monoethylenically unsaturated monomers [e.g., pinene, limonene, and indene]; and polyethylenic vinyl polymerizable monomers [e.g., cyclopentadiene, bicyclopentadiene, and ethylidene norbornene]; etc.

When an additional vinyl monomer (a') is contained as a constitutional unit, the content (mol%) of the additional vinyl monomer (a') unit is preferably 0.01 to 5 based on the number of moles of the water-soluble vinyl monomer (a1) unit and the vinyl monomer (a2) unit, more preferably 0.05 to 3, even more preferably 0.08 to 2, and particularly preferably 0.1 to 1.5. From the viewpoint of absorption characteristics, etc., it is most preferable that the content of the additional vinyl monomer (a') unit be 0 mol%.

The crosslinking agent (a3) is not particularly restricted and conventional crosslinking agents (e.g., Japanese Patent No. 3648553, JP-A-2003-165883, JP-A-2005-75982, and JP-A-2005-95759), etc. can be used. Among these, preferred from the viewpoint of absorption characteristics, etc. are crosslinking agents having two or more ethylenically unsaturated groups and crosslinking agents having two or more epoxy groups, more preferred are poly(meth)allyl ethers of polyols having 2 to 10 carbon atoms and diglycidyl ethers having a degree of polymerization of 2 to 10 of polyethylene glycol, particularly preferred are triallyl cyanurate, triallyl isocyanurate, tetraallyloxyethane, pentaerythritol triallyl ether, and diglycidyl ethers having a degree of polymerization of 2 to 5 of polyethylene glycol, and most preferred are pentaerythritol triallyl ether and ethylene glycol diglycidyl ether.

The content (mol%) of the crosslinking agent (a3) units is 0.001 to 5 based on the number of moles of the water-soluble vinyl monomer (a1) units and the vinyl monomer (a2) units, more preferably 0.005 to 3, and particularly preferably 0.01 to 1. When the content is within such ranges, absorption characteristics are further improved.

The crosslinked polymer particles (A) may be of a single species or alternatively may be a mixture of two or more species .

The crosslinked polymer particles (A) can be produced in the same way as in conventional aqueous solution polymerization (adiabatic polymerization, thin film polymerization, spray polymerization, etc. disclosed in JP-A-55-133413, etc.) or in conventional reverse- phase suspension polymerization (the methods disclosed in JP-B-54-30710, JP-A-56-26909, JP-A-1-5808, etc.). Of such polymerization methods, a solution polymerization method is preferable because it does not require use of organic solvents, etc., and is advantageous in the production cost aspect, and an aqueous solution polymerization method and a reverse-phase suspension polymerization method are more preferable in terms of tangleability with the nonwoven fabric (B).

The hydrous gel to be produced by polymerization, which is a gel-like material composed of the crosslinked polymer (A) and water, may be chopped, if necessary. The size (longest diameter) of the chopped gel is preferably from 50 μm to 10 cm, more preferably from 100 μm to 2 cm, and particularly preferably from 1 mm to 1 cm. When the size is within such ranges, dryability during a drying step is further improved.

Chopping can be carried out by a conventional method and chopping can be done by using a chopping machine (e.g., a Bex Mill, a rubber chopper, a Pharma Mill, a mincing machine, an impact type pulverizer, and a roll type pulverizer), etc.

When a solvent (organic solvents, water, etc.) is used for polymerization, it is preferred to distill off the solvent after the polymerization. When an organic solvent is contained in the solvent, the content (% by weight) of the organic solvent after distillation, based on the weight of absorbent resin particles, is preferably 0 to 10, more preferably 0 to 5, particularly preferably 0 to 3, and most preferably 0 to 1. When the content is within such ranges, the absorption performance (especially, water retention capacity) of the crosslinked polymer particles (A) is further improved.

When water is contained in the solvent, the content (% by weight) of water after distillation, based on the weight of the crosslinked polymer, is preferably 0 to 20, more preferably 1 to 10, particularly preferably 2 to 9, and most preferably 3 to 8. When the content is within such ranges, absorption performance and the breakability of the crosslinked polymer particles (A) after drying are further improved.

The contents of an organic solvent and water can be determined from the weight loss of a measurement sample before and after heating which is performed with an infrared moisture content analyzer {e.g., JE400 manufactured by Kett Electric Laboratory: $120 \pm 5$°C, 30 minutes, atmosphere humidity before heating $50 \pm 10$ %RH, lamp specification: 100 V, 40 W}.

As a method of distilling off a solvent (including water), a method of distilling (drying) it with hot blast having a temperature of from 80 to 230°C, a thin film drying method using, e.g., a drum dryer heated at 100 to 230°C, a (heating) reduced pressure drying method, a freeze drying method, a drying method using infrared radiation, decantation, filtration, etc. can be applied.

The crosslinked polymer can be pulverized after drying. The method of pulverization is not particularly restricted, and ordinary pulverizing apparatuses {e.g., a hammer type pulverizer, an impact type pulverizer, a roll type pulverizer, and a jet stream type pulverizer}, etc. can be used. The pulverized crosslinked polymer can be adjusted in its particle size by screening, etc. according to need.

The weight average particle diameter ($\mu$m) of the crosslinked polymer particles (A) having been screened according to need is preferably 100 to 800, more preferably 200 to 700, even more preferably 250 to 600, particularly preferably 300 to 500, and most preferably 350 to 450. In these ranges, the absorption performance is further improved and tangleability with the nonwoven fabric (B) is also improved, resulting in good shape retention.

The weight average particle diameter is measured by the method disclosed in Perry's Chemical Engineers' Handbook, Sixth Edition (McGraw-Hill Book Company, 1984, page 21) by using a RO-TAP sieve shaker and standard sieves (JIS Z8801-1:2006). Specifically, JIS standard sieves are combined, for example, in the order of 1000 $\mu$m, 850 $\mu$m, 710 $\mu$m, 500 $\mu$m, 425 $\mu$m, 355 $\mu$m, 250 $\mu$m, 150 $\mu$m, 125 $\mu$m, 75 $\mu$m, 45 $\mu$m, and a bottom tray when viewed from the top. About 50 g of particles to be measured are put on the top sieve and then shaken for five minutes by a RO-TAP sieve shaker. Then, the measured particles received on the respective sieves and the bottom tray are weighed and the weight fractions of the particles on the respective sieves are calculated with the total weight of the particles considered to be 100% by weight. The calculated values are plotted on a logarithmic probability sheet {taking the size of openings of a sieve (particle diameter) as an abscissa and the weight fraction as an ordinate} and then a line connecting the respective points is drawn. Subsequently, a particle diameter that corresponds to a weight fraction of 50% by weight is determined and this is defined as a weight average particle diameter.

Since a smaller content of particulates results in better absorption performance, the content of particulates being 106 $\mu$m or less in size (preferably being 150 $\mu$m or less in size) accounted for of all particles is preferably 3% by weight or less, and more preferably 1% by weight or less. The content of particulates can be determined using a plot produced when determining the weight average particle diameter.

The apparent density (g/ml) of the crosslinked polymer particles (A) is preferably from 0.50 to 0.75, more preferably from 0.54 to 0.73, and particularly preferably from 0.56 to 0.64. When the apparent density is within such ranges, the absorption performance is further improved. The apparent density is measured at 25°C in accordance with JIS K7365:1999.

The shape of the crosslinked polymer particles (A) is not particularly restricted and may be an irregularly pulverized form, a scaly form, a pearl-like form, a rice grain form, etc. Of these, an irregularly pulverized form and a pearl granule form are preferred from the viewpoints of good tanglement with the nonwoven fabric (B) and the hydrophilic fibers (c) and retention of the shape of the absorber.

The crosslinked polymer particles (A) may be subjected to a surface crosslinking treatment with a surface crosslinking agent according to need. As the surface crosslinking agent, conventional surface crosslinking agents (e.g., multivalent glycidyl ethers, polyhydric alcohols, polyamines, multivalent aziridines, polyisocyanates, ethylene carbonate, silane coupling agents, and multivalent metals disclosed in JP-A-59-189103, JP-A-58-180233, JP-A-61-16903, JP-A-61-211305, JP-A-61-252212, JP-A-51-136588, and JP-A-61-257235), etc. can be used. Of these surface crosslinking agents, multivalent glycidyl ethers, polyhydric alcohols, and polyamines are preferred, multivalent glycidyl ethers and polyhydric alcohols are more preferred, multivalent glycidyl ethers are particularly preferred, and ethylene glycol diglycidyl ether is most preferred from the viewpoints of economical efficiency and absorption characteristics.

When a surface crosslinking treatment is performed, the amount (% by weight) of the surface crosslinking agent used is not particularly restricted because it can be varied depending upon the type of the surface crosslinking agent, the conditions for crosslinking, target performance, etc., however, from the viewpoint of absorption characteristics, etc., it is preferably from 0.001 to 3, more preferably from 0.005 to 2, and particularly preferably from 0.01 to 1, based on the weight of the water-soluble vinyl monomer (a1), the vinyl monomer (a2), and the crosslinking agent (a3).

When a surface crosslinking treatment is performed, conventional methods (disclosed in, for example, Japanese Patent No. 3648553, JP-A-2003-165883, JP-A-2005-75982, and JP-A-2005-95759) can be used as the method of the surface crosslinking treatment.

It is preferred from the viewpoint of appropriate absorption rate pattern that the crosslinked polymer particles (A) further contain a hydrophobic substance (a4). Examples of the hydrophobic substance (a4) include hydrophobic substances containing a hydrocarbon group (a4-1), hydrophobic substances containing a hydrocarbon group having a fluorine atom (a4-2), and hydrophobic substances having a polysiloxane structure (a4-3).

Examples of the hydrophobic substances containing a hydrocarbon group (a4-1) include polyolefin resins, polyolefin resin derivatives, polystyrene resins, polystyrene resin derivatives, waxes, long chain fatty acid esters, long chain fatty acids and salts thereof, long chain aliphatic alcohols, long chain aliphatic amides, and mixtures of two or more members of the foregoing.

As the polyolefin resin, there can be enumerated polymers having a weight average molecular weight of 1000 to 1000000 and containing an olefin having 2 to 4 carbon atoms (e.g., ethylene, propylene, isobutylene, and isoprene) as an essential constituent monomer (the content of the olefin is at least 50% by weight based on the weight of the polyolefin resin) (e.g., polyethylene, polypropylene, polyisobutylene, poly(ethylene-isobutylene), and isoprene).

As the polyolefin resin derivative, there can be enumerated polymers having a weight average molecular weight of 1000 to 1000000 and being obtained by introducing a carboxy group (-COOH), 1,3-oxo-2-oxapropylene (-COOCO-), or the like into a polyolefin resin (e.g., thermally degraded polyethylene, thermally degraded polypropylene, maleic acid-modified polyethylene, chlorinated polyethylene, maleic acid-modified polypropylene, an ethylene-acrylic acid copolymer, an ethylene-maleic anhydride copolymer, an isobutylene-maleic anhydride copolymer, maleated polybutadiene, an ethylene-vinyl acetate copolymer, and a maleated product of an ethylene-vinyl acetate copolymer). As the polystyrene resin, there can be enumerated, for example, polymers having a weight average molecular weight of 1000 to 1000000.

As the polystyrene resin derivative, there can be enumerated, for example, polymers having a weight average molecular weight of 1000 to 1000000 and containing styrene as an essential constituent monomer (the content of styrene is at least 50% by weight based on the weight of the polystyrene derivative) (e.g., a styrene-maleic anhydride copolymer, a styrene-butadiene copolymer, and a styrene-isobutylene copolymer).

As the wax, there can be enumerated waxes having a melting point of 50 to 200°C (e.g., paraffin wax, bees wax, carnauba wax, and beef tallow).

As the long chain fatty acid ester, there can be enumerated esters of a fatty acid having 8 to 30 carbon atoms with an alcohol having 1 to 12 carbon atoms (e.g., methyl laurate, ethyl laurate, methyl stearate, ethyl stearate, methyl oleate, ethyl oleate, glycerol monolaurate, glycerol monostearate, glycerol monooleate, pentaerythritol monolaurate, pentaerythritol monostearate, pentaerythritol monooleate, sorbitol monolaurate, sorbitol monostearate, sorbitol monooleate, sucrose monopalmitate, sucrose dipalmitate, sucrose tripalmitate, sucrose monostearate, sucrose distearate, sucrose tristearate, and beef tallow).

As the long chain fatty acid and the salt thereof, there can be enumerated fatty acids having 8 to 30 carbon atoms (e.g., lauric acid, palmitic acid, stearic acid, oleic acid, dimer acid, and behenic acid) . As the salt thereof, there can be enumerated salts with zinc, calcium, magnesium or aluminum (hereinafter abbreviated as Zn, Ca, Mg, and Al) (e.g., Ca palmitate, Al palmitate, Ca stearate, Mg stearate, and Al stearate).

As the long chain aliphatic alcohol, there can be enumerated aliphatic alcohols having 8 to 30 carbon atoms (e.g., lauryl alcohol, palmityl alcohol, stearyl alcohol, and oleyl alcohol) . From the viewpoint of the leakage resistance of the absorbent article, etc., palmityl alcohol, stearyl alcohol, and oleyl alcohol are preferred, and stearyl alcohol is more preferred.

As the long chain aliphatic amide, there can be enumerated amidated products of a long chain aliphatic primary amine having 8 to 30 carbon atoms with a carboxylic acid having a hydrocarbon group having 1 to 30 carbon atoms, amidated products of ammonia or a primary amine having 1 to 7 carbon atoms with a long chain fatty acid having 8 to 30 carbon atoms, amidated products of a long chain aliphatic secondary amine having at least one aliphatic chain having 8 to 30 carbon atoms with a carboxylic acid having 1 to 30 carbon atoms, and amidated products of a secondary amine having two aliphatic hydrocarbon groups having 1 to 7 carbon atoms with a long chain fatty acid having 8 to 30 carbon atoms.

The amidated products of a long chain aliphatic primary amine having 8 to 30 carbon atoms with a carboxylic acid having a hydrocarbon group having 1 to 30 carbon atoms are classified into one obtained by reacting a primary amine with a carboxylic acid in a ratio of 1 : 1 and one obtained by reacting a primary amine with a carboxylic acid in a ratio of 1 : 2. As the one obtained by reacting a primary amine with a carboxylic acid in a ratio of 1 : 1, there can be enumerated, for example, acetic acid N-octylamide, acetic acid N-hexacosylamide, heptacosanoic acid N-octylamide, and heptacosanoic acid N-hexacosylamide. As the one obtained by reacting a primary amine with a carboxylic acid in a ratio of 1 : 2, there can be enumerated, for example, diacetic acid N-octylamide, diacetic acid N-hexacosylamide, diheptacosanoic acid N-octylamide, and diheptacosanoic acid N-hexacosylamide. In the case of the one obtained by reacting a primary amine with a carboxylic acid in a ratio of 1 : 2, the carboxylic acids to be used may be either the same or different.

The amidated products of ammonia or a primary amine having 1 to 7 carbon atoms with a long chain fatty acid having 8 to 30 atoms are classified into one obtained by reacting ammonia or a primary amine with a carboxylic acid in a ratio of 1 : 1 and one obtained by reacting ammonia or a primary amine with a carboxylic acid in a ratio of 1 : 2. As the one obtained by reacting ammonia or a primary amine with a carboxylic acid in a ratio of 1 : 1, there can be enumerated, for example, nonanoic acid amide, nonanoic acid methylamide, nonanoic acid N-heptylamide, heptacosanoic acid amide, heptacosanoic acid N-methylamide, heptacosanoic acid N-heptylamide, and heptacosanoic acid N-hexacosylamide. As the one obtained by reacting ammonia or a primary amine with a carboxylic acid in a ratio of 1 : 2, there can be enumerated, for example, dinonanoic acid amide, dinonanoic acid N-methylamide, dinonanoic acid N-heptylamide, dioctadecanoic acid amide, dioctadecanoic acid N-ethylamide, dioctadecanoic acid N-heptylamide, diheptacosanoic acid amide, diheptacosanoic acid N-methylamide, diheptacosanoic acid N-heptylamide, and diheptacosanoic acid N-hexacosylamide. In the case of the one obtained by reacting ammonia or a primary amine with a carboxylic acid in a ratio of 1 : 2, the carboxylic acids to be used may be either the same or different.

As the amidated products of a long chain aliphatic secondary amine having at least one aliphatic chain having 8 to 30 carbon atoms with a carboxylic acid having 1 to 30 carbon atoms, there can be enumerated, for example, acetic acid N-methyloctylamide, acetic acid N-methylhexacosylamide, acetic acid N-octylhexacosylamide, acetic acid N-dihexacosylamide, heptacosanoic acid N-methyloctylamide, heptacosanoic acid N-methylhexacosylamide, heptacosanoic acid N-octylhexacosylamide, and heptacosanoic acid N-dihexacosylamide.

As the amidated products of a secondary amine having two aliphatic hydrocarbon groups having 1 to 7 carbon atoms with a long chain fatty acid having 8 to 30 carbon atoms, there can be enumerated, for example, nonanoic acid N-dimethylamide, nonanoic acid N-methylheptylamide, nonanoic acid N-diheptylamide, heptacosanoic acid N-dimethylamide, heptacosanoic acid N-methylheptylamide, and heptacosanoic acid N-diheptylamide.

Examples of the hydrophobic substances containing a hydrocarbon group with a fluorine atom (C2) include perfluoroalkanes, perfluoroalkenes, perfluoroaryls, perfluoroalkyl ethers, perfluoroalkyl carboxylic acids, perfluoroalkyl alcohols, and mixtures of two or more members of the foregoing.

As the perfluoroalkanes, there can be enumerated alkanes having 4 to 42 fluorine atoms and 1 to 20 carbon atoms (e.g., trifluoromethane, pentafluoroethane, pentafluoropropane, heptafluoropropane, heptafluorobutane, nonafluorohexane, tridecafluorooctane, and heptadecafluorododecane).

As the perfluoroalkenes, there can be enumerated alkenes having 4 to 42 fluorine atoms and 2 to 20 carbon atoms (e.g., trifluoroethylene, pentafluoropropene, trifluoropropene, heptafluorobutene, nonafluorohexene, tridecafluorooctene, and heptadecafluorododecene).

As the perfluoroaryls, there can be enumerated aryls having 4 to 42 fluorine atoms and 6 to 20 carbon atoms (e.g., trifluorobenzene, pentafluorotoluene, trifluoronaphthalene, heptafluorobenzene, nonafluoroxylene, tridecafluorooctylbenzene, and heptadecafluorododecylbenzene).

As the perfluoroalkyl ethers, there can be enumerated ethers having 2 to 82 fluorine atoms and 2 to 40 carbon atoms (e.g., ditrifluoromethyl ether, dipentafluoroethyl ether, dipentafluoropropyl ether, diheptafluoropropyl ether, diheptafluorobutyl ether, dinonafluorohexyl ether, ditridecafluorooctyl ether, and diheptadecafluorododecyl ether).

As the perfluoroalkylcarboxylic acids, there can be enumerated carboxylic acids having 3 to 41 fluorine atoms and 1 to 21 carbon atoms {e.g., pentafluoroethanoic acid, pentafluoropropanoic acid, heptafluoropropanoic acid, heptafluorobutanoic acid, nonafluorohexanoic acid, tridecafluorooctanoic acid, heptadecafluorododecanoic acid, and metal (alkali metal, alkaline earth metal, etc.) salts thereof}.

As the perfluoroalkyl alcohols, there can be enumerated, for example, alcohols having 3 to 41 fluorine atoms and 1 to 20 carbon atoms (e.g., pentafluoroethanol, pentafluoropropanol, heptafluoropropanol, heptafluorobutanol, nonafluorohexanol, tridecafluorooctanol, and heptadecafluorododecanol) and ethylene oxide adducts of the alcohols (1 to 20 mol of ethylene oxide per mol of the alcohol).

As the mixtures of two or more thereof, there can be enumerated mixtures of a perfluoroalkylcarboxylic acid and a perfluoroalkyl alcohol (e.g., a mixture of pentafluoroethanoic acid and pentafluoroethanol).

Examples of the hydrophobic substances having a polysiloxane structure (a4-3) include polydimethylsiloxanes, polyether-modified polysiloxanes {e.g., polyoxyethylene-modified polysiloxanes and poly(oxyethylene-oxypropylene)-modified polysiloxanes}, carboxy-modified polysiloxanes, epoxy-modified polysiloxanes, amino-modified polysiloxanes, alkoxy-modified polysiloxanes, and mixtures thereof.

The position of the organic group (modifying group) of a modified silicone {e.g., polyether-modified polysiloxanes, carboxy-modified polysiloxanes, epoxy-modified polysiloxanes, and amino-modified polysiloxanes} is not particularly restricted and it may be on a side chain of a polysiloxane, at both terminals of a polysiloxane, at one terminal of a polysiloxane, and both of on a side chain and at both terminals of a polysiloxane. Of these, on a side chain of a polysiloxane and both of a side chain and both terminals of a polysiloxane are preferred, and both of on a side chain and at both terminals of a polysiloxane are more preferred, from the viewpoint of absorption characteristics, etc.

Examples of the organic group (modifying group) of polyether-modified polysiloxanes include groups containing a polyoxyethylene group or a poly(oxyethylene-oxypropylene) group. The content (number) of the oxyethylene groups and/or the oxypropylene groups contained in the polyether-modified polysiloxane is preferably 2 to 40, more preferably 5 to 30, particularly preferably 7 to 20, and most preferably 10 to 15 per molecule of the polyether-modified polysiloxane. When the content is within such ranges, absorption characteristics are further improved. When oxyethylene groups and oxypropylene groups are contained, the content (% by weight) of the oxyethylene groups is preferably 1 to 30, more preferably 3 to 25, and particularly preferably 5 to 20 based on the weight of the polysiloxane. When the content is within such ranges, absorption characteristics are further improved.

The polyether-modified polysiloxanes can easily be obtained from the market and can preferably be exemplified by the following commercially available products (modified position, type of oxyalkylene).

• Products manufactured by Shin-Etsu Chemical Co., Ltd.

KF-945 (side chain, oxyethylene and oxypropylene), KF-6020 (side chain, oxyethylene and oxypropylene), X-22-6191 (side chain, oxyethylene and oxypropylene), X-22-4952 (side chain, oxyethylene and oxypropylene), X-22-4272 (side chain, oxyethylene and oxypropylene), and X-22-6266 (side chain, oxyethylene and oxypropylene).

• Products manufactured by Dow Corning Toray Co., Ltd.

FZ-2110 (both terminals, oxyethylene and oxypropylene), FZ-2122 (both terminals, oxyethylene and oxypropylene), FZ-7006 (both terminals, oxyethylene and oxypropylene), FZ-2166 (both terminals, oxyethylene and oxypropylene), FZ-2164 (both terminals, oxyethylene and oxypropylene), FZ-2154 (both terminals, oxyethylene and oxypropylene), FZ-2203 (both terminals, oxyethylene and oxypropylene), and FZ-2207 (both terminals, oxyethylene and oxypropylene).

Examples of the organic group (modifying group) of carboxy-modified polysiloxanes include groups containing a carboxy group, examples of the organic group (modifying group) of epoxy-modified polysiloxanes include groups containing an epoxy group, and examples of the organic group (modifying group) of amino-modified polysiloxanes include groups containing an amino group (primary, secondary, or tertiary amino group). The content (g/mol) of the organic group (modifying group) of such a modified silicone is preferably 200 to 11000, more preferably 600 to 8000, and particularly preferably 1000 to 4000, expressed by carboxy equivalent, epoxy equivalent, or amino equivalent. When the content is within such ranges, absorption characteristics are further improved. Herein, the carboxy equivalent is measured in accordance with "16. Total Acid Value Test" of JIS C2101:1999. The epoxy equivalent is determined in accordance with JIS K7236:2001. The amino equivalent is measured in accordance with "8. Potentiometric Titration Method (base number; hydrochloric acid method)" of JIS K2501:2003.

The carboxy-modified polysiloxanes can easily be obtained from the market and can preferably be exemplified by the following commercially available products (modified position, carboxy equivalent (g/mol)).

• Products manufactured by Shin-Etsu Chemical Co., Ltd.

X-22-3701E (side chain, 4000), X-22-162C (both terminals, 2300), and X-22-3710 (one terminal, 1450).

• Products manufactured by Dow Corning Toray Co., Ltd.

BY 16-880 (side chain, 3500), BY 16-750 (both terminals, 750), BY 16-840 (side chain, 3500), and SF8418 (side chain, 3500).

The epoxy-modified polysiloxanes can easily be obtained from the market and can preferably be exemplified by the following commercially available products (modified position, epoxy equivalent).

• Products manufactured by Shin-Etsu Chemical Co., Ltd.

X-22-343 (side chain, 525), KF-101 (side chain, 350), KF-1001 (side chain, 3500), X-22-2000 (side chain, 620), X-22-2046 (side chain, 600), KF-102 (side chain, 3600), X-22-4741 (side chain, 2500), KF-1002 (side chain, 4300), X-22-3000T (side chain, 250), X-22-163 (both terminals, 200), KF-105 (both terminals, 490), X-22-163A (both terminals, 1000), X-22-163B (both terminals, 1750), X-22-163C (both terminals, 2700), X-22-169AS (both terminals, 500), X-22-169B (both terminals, 1700), X-22-173DX (one terminal, 4500), and X-22-9002 (side chain/both terminals, 5000).

• Products manufactured by Dow Corning Toray Co., Ltd.

FZ-3720 (side chain, 1200), BY 16-839 (side chain, 3700), SF 8411 (side chain, 3200), SF 8413 (side chain, 3800), SF 8421 (side chain, 11000), BY 16-876 (side chain, 2800), FZ-3736 (side chain, 5000), BY 16-855D (side chain, 180), and BY 16-8 (side chain, 3700).

The amino-modified silicones can easily be obtained from the market and can preferably be exemplified by the following commercially available products (modified position, amino equivalent).

• Products manufactured by Shin-Etsu Chemical Co., Ltd.

KF-865 (side chain, 5000), KF-864 (side chain, 3800), KF-859 (side chain, 6000), KF-393 (side chain, 350), KF860 (side chain, 7600), KF-880 (side chain, 1800), KF-8004 (side chain, 1500), KF-8002 (side chain, 1700), KF-8005 (side chain, 11000), KF-867 (side chain, 1700), X-22-3820W (side chain, 55000), KF-869 (side chain, 8800), KF-861 (side chain, 2000), X-22-3939A (side chain, 1500), KF-877 (side chain, 5200), PAM-E (both terminals, 130), KF-8010 (both terminals, 430), X-22-161A (both terminals, 800), X-22-161B (both terminals, 1500), KF-8012 (both terminals, 2200), KF-8008 (both terminals, 5700), X-22-1660B-3 (both terminals 2200), KF-857 (side chain, 2200), KF-8001 (side chain, 1900), KF-862 (side chain, 1900), and X-22-9192 (side chain, 6500).

• Products manufactured by Dow Corning Toray Co., Ltd.

FZ-3707 (side chain, 1500), FZ-3504 (side chain, 1000), BY 16-205 (side chain, 4000), FZ-3760 (side chain, 1500), FZ-3705 (side chain, 4000), BY 16-209 (side chain, 1800), FZ-3710 (side chain, 1800), SF 8417 (side chain, 1800), BY 16-849 (side chain, 600), BY 16-850 (side chain, 3300), BY 16-879B (side chain, 8000), BY 16-892 (side chain, 2000), FZ-3501 (side chain, 3000), FZ-3785 (side chain, 6000), BY 16-872 (side chain, 1800), BY 16-213 (side chain, 2700), BY 16-203 (side chain, 1900), BY 16-898 (side chain, 2900), BY 16-890 (side chain, 1900), BY 16-893 (side chain, 4000), FZ-3789 (side chain, 1900), BY 16-871 (both terminals, 130), BY 16-853C (both terminals, 360), and BY 16-853U (both terminals, 450).

As the mixtures thereof, there can be enumerated, for example, mixtures of a polydimethylsiloxane and a carboxyl-modified polysiloxane, and mixtures of a polyether-modified polysiloxane and an amino-modified polysiloxane.

The viscosity (mPa•s, 25°C) of the hydrophobic substance having a polysiloxane structure is preferably 10 to 5000 mPa•s, more preferably 15 to 3000 mPa•s, and particularly preferably 20 to 1500 mPa•s. When the viscosity is within such ranges, absorption characteristics are further improved. Herein, the viscosity is measured in accordance with JIS Z8803-1991 "Viscosity of Liquid" 9. The viscosimetry by means of a circular cone and circular cone-flat plate type rotary viscometer {for example, the viscosity is measured by means of an E type viscometer (RE80L manufactured by Toki Sangyo Co., Ltd., a circular cone having a radius of 7 mm and an angle of $5.24 \times 10^{-2}$ rad) whose temperature is adjusted to $25.0 \pm 0.5°C$}.

The HLB value of the hydrophobic substance (a4) is preferably 1 to 10, more preferably 2 to 8, and particularly preferably 3 to 7. When the HLB value is within such ranges, the leakage resistance of the absorbent article is further improved. The HLB value means a value of hydrophilicity-hydrophobicity balance (HLB) and is determined by the ODA method (Shin-Kaimen Kassei Zai Nyumon, page 197, Takehiko Fujimoto, published by Sanyo Chemical Industries Ltd., 1981).

From the viewpoint of the leakage resistance of an absorbent article, the hydrophobic substance (a4) is preferably a hydrophobic substance containing a hydrocarbon group (a4-1), more preferably a long chain fatty acid ester, a long chain fatty acid and a salt thereof, a long chain aliphatic alcohol, and a long chain aliphatic amide, further preferably sorbitol stearate, sucrose stearate, stearic acid, Mg stearate, Ca stearate, Zn stearate, and Al stearate, particularly preferably sucrose stearate and Mg stearate, and most preferably sucrose distearate.

The hydrophobic substance (a4) may be present at any portion of the crosslinked polymer particles.

From the viewpoints of the skin irritation resistance of an absorbent article and the leakage resistance of an absorbent article, the content (% by weight) of the hydrophobic substance (a4) in the inside of the crosslinked polymer particles is usually 0.01 to 10.0, preferably 0.01 to 5.0, more preferably 0.05 to 2.0, and particularly preferably 0.1 to 1.0, based on the weight of the crosslinked polymer particles (A).

From the viewpoints of the skin irritation resistance of an absorbent article and the leakage resistance of an absorbent article, the content (% by weight) of the hydrophobic substance (a4) existing on the surface of the crosslinked polymer particles (A) is usually 0.001 to 1.0, preferably 0.005 to 0.5, more preferably 0.01 to 0.3, and particularly preferably 0.01 to 0.1, based on the weight of the crosslinked polymer (A1).

The content of the hydrophobic substance existing on the surface is measured by the method described below.

The content of the hydrophobic substance existing in the inside is defined to be the amount obtained by subtracting the content of the hydrophobic substance on the surface from the total amount of the hydrophobic substance added.

<Method of measuring content of hydrophobic substance (a4) on surface>

To a glass-made eggplant-shaped flask fitted with a cooling tube are added 100 parts by weight of absorbent resin particles and 300 parts by weight of an organic solvent (an organic solvent capable of dissolving at least 0.01 parts by weight of the hydrophobic substance (a4) in 100 parts by weight of the organic solvent at 25°C to 110°C; the temperature at which the hydrophobic substance can be dissolved is regarded as dissolution temperature) and the resulting mixture is allowed to stand at the dissolution temperature for 24 hours, so that an extraction solution of the hydrophobic substance is obtained. After the extraction solution is filtered using a filter paper and is collected into a glass-made eggplant-shaped flask weighed beforehand, the solvent is evaporated with a rotary evaporator and then the flask is weighed. The amount of the extracted evaporation residue is determined by subtracting the weight of the eggplant-shaped flask weighed beforehand from the weight after the evaporation of the filtrate.

The same operation as above is further repeated twice using the extracted sample remaining on the filter paper, and the total amount of the extracted evaporation residue collected via the three extractions is regarded as the content (% by weight) of the hydrophobic substance (a4) on the surface.

The structure in which the hydrophobic substance (a4) exists in the inside of the crosslinked polymer particles and a part thereof is present on the surface can be produced by the following methods.

Production method (1): a method of mixing and kneading absorbent resin particles with the hydrophobic substance (a4) and a hydrous gel of a crosslinked polymer (A1);

Production method (2): a method of obtaining a hydrous gel of a crosslinked polymer (A1) by polymerizing constitutional units into absorbent resin particles in the presence of the hydrophobic substance (a4).

In the production method (1), as to the shape of the hydrophobic substance (a4), one processed into a pulverized form, a bead form, a rod-like form, or a fibrous form can be used. From the viewpoint of the leakage resistance of an absorbent article, etc., a pulverized form or a bead form is preferable, and a bead form is more preferable. The volume average particle diameter ($\mu$m) of the hydrophobic substance (a4) is preferably 0.5 to 100, more preferably 1 to 30, and particularly preferably 2 to 20.

The method of mixing the crosslinked polymer (A1) with the hydrophobic substance (a4) is not restricted if they can be mixed so that the hydrophobic substance (a4) may be present in the inside of the crosslinked polymer (A1). Particularly, the hydrophobic substance (a4) is preferably mixed not with the crosslinked polymer (A1) having been dried but with a hydrous gel of (A1) or a polymerized liquid of (A1), and is more preferably mixed with a hydrous gel of (A1). As to mixing, it is preferred to mix them uniformly so that they may be kneaded.

When the crosslinked polymer (A1) is produced by an aqueous solution polymerization method, although there is no particular restriction with respect to the timing of mixing and kneading the hydrophobic substance (a4) with (A1), it can be, for example, during a polymerization step {(A1) is produced in the presence of (a4)}, just after a polymerization step, during mincing a hydrous gel, or during drying of a hydrous gel. Among these, from the viewpoint of leakage resistance of an absorbent article, etc., just after a polymerization step and during a step of mincing a hydrous gel are preferable, and during a step of mincing a hydrous gel is more preferable. When the hydrophobic substance (a4) is a long chain fatty acid salt, the long chain fatty acid salt itself is usually used, however, a long chain fatty acid and a metal hydroxide may be mixed and added during the addition step or alternatively they may be added separately.

When the crosslinked polymer (A1) is produced by a reverse-phase suspension polymerization method or an emulsion polymerization method, although there is no particular restriction with respect to the timing of mixing the hydrophobic substance (a4) with (A1), it can be, for example, during a polymerization step {(A1) is produced in the presence of (a4)}, just after a polymerization step, during a dehydration step (during a step of dehydrating to a moisture content of about 10% by weight), just after a dehydration step, during a step of evaporating the organic solvent used for polymerization, or during drying of a hydrous gel. Among these, from the viewpoint of leakage resistance of an absorbent article, etc., during a polymerization step, just after a polymerization step, during a dehydration step, just after a dehydration step, and during a step of evaporating the organic solvent used for polymerization are preferred, and more preferred are during a polymerization step and just after a polymerization step.

When the mixing is carried out during mincing or drying of a hydrous gel, such an ordinary apparatus as a Bex Mill, a rubber chopper, a Pharma Mill, a mincing machine, an impact type pulverizer, and a roll type pulverizer can be used as a mixing apparatus. When the mixing is carried out in a polymerization liquid, an apparatus with a relatively high stirring force, such as a homomixer and a biomixer, can be used. When the mixing is carried out during drying of a hydrous gel, such a kneading apparatus as an SV mixer can also be used.

The mixing temperature (°C) can appropriately be adjusted by the step of adding the hydrophobic substance (a4). For example, the mixing temperature (°C) in the case of adding and mixing just after a polymerization step and during a step of mincing a hydrous gel is preferably 20 to 100, more preferably 40 to 90, and particularly preferably 50 to 80. Within such ranges, it becomes easier to perform uniform mixing and absorption characteristics are further improved.

In the production method (2) of producing a crosslinked polymer (A1) in the presence of a hydrophobic substance (a4), it is preferable to uniformly dissolve or emulsify (disperse) the hydrophobic substance (a4) in a polymerization liquid of the crosslinked polymer (A1). When it is difficult to render the hydrophobic substance (a4) uniform, it can be rendered uniform during the step of mincing the hydrous gel. This can be performed while (a4) is allowed to deposit along with the progress of the polymerization of (A1). The polymerization method is the same as that of the case of the crosslinked polymer (A1) except performing polymerization in the presence of hydrophobic substance (a4).

The hydrophobic substance (a4) can be used in a form of having been dissolved and/or emulsified in water and/or a volatile solvent (no emulsifier is used). As the volatile solvent, one having a vapor pressure (Pa) at 20°C of from 0.13 to 5.3 is preferred, one having a vapor pressure of from 0.15 to 4.5 is more preferred, and one having a vapor pressure of from 0.23 to 3.8 is particularly preferred, from the viewpoint of ease of removal thereof, etc.

Examples of the volatile solvent include alcohols having 1 to 3 carbon atoms (e.g., methanol, ethanol, and isopropanol), hydrocarbons having 5 to 8 carbon atoms (e.g., pentane, hexane, cyclohexane, and toluene), ethers having 2 to 4 carbon atoms (e.g., dimethyl ether, diethyl ether, and tetrahydrofuran), ketone having 3 to 4 carbon atoms (e.g., acetone and methyl ethyl ketone), and esters having 3 to 5 carbon atoms (e.g., ethyl formate, ethyl acetate, isopropyl acetate, and diethyl carbonate). When water and/or a volatile solvent are used, the amount (% by weight) of them used is preferably 1 to 900 based on the weight of the hydrophobic substance (a4), more preferably 5 to 700, and particularly preferably 10 to 400. When water and a volatile solvent are used, the amount (% by weight) of the water used is preferably 50 to 98 based on the combined weight of the water and the volatile solvent, more preferably 60 to 95, and particularly preferably 70 to 90.

The crosslinked polymer particles (A) can further be coated with an inorganic powder (a5) on their surface. As a preferable inorganic powder (a5), there are enumerated, for example, glass, a silica gel, a silica sol, silica, clay, a carbon fiber, kaolin, talc, mica, bentonite, sericite, asbestos, and Shirasu. Preferred of the inorganic powders (a5) area silica sol, silica, and talc.

As to the shape of the inorganic powder (a5), any of an indefinite form (pulverized form), a spherical form, a film-like form, a rod-like form, and a fibrous form is available, but an indefinite form (pulverized form) or a spherical form is preferable, and a spherical form is more preferable.

The content (% by weight) of the inorganic powder (a5) is preferably 0.01 to 3.0 based on the weight of the crosslinked polymer (A1), more preferably 0.05 to 1.0, even more preferably 0.07 to 0.8, particularly preferably 0.10 to 0.6, and most preferably 0.15 to 0.5. Within such ranges, the skin irritation resistance of an absorbent article is further improved.

The crosslinked polymer particles (A) can also contain other additives {for example, antiseptics, antifungal agents, antibacterial agents, antioxidants, UV absorbents, coloring agents, aromatics, deodorants, and organic fibrous materials known in the art (JP-A-2003-225565 and JP-A-2006-131767)}. When such an additive is contained, the content (% by weight) of the additive is preferably 0.001 to 10 based on the weight of the crosslinked polymer (A1), more preferably 0.01 to 5, particularly preferably 0.05 to 1, and most preferably 0.1 to 0.5.

The crosslinked polymer particles (A) are preferably crosslinked polymer particles that absorb physiological saline 40 times their own weight in 40 to 150 seconds (more preferably in 55 to 120 seconds, and particularly preferably in 65 to 110 seconds).

Within such ranges, the skin irritation resistance of an absorbent article is further improved. By adjusting the content of the hydrophobic substance (a4) and the average particle diameter and the apparent density of the crosslinked polymer to the aforementioned preferable ranges, the absorption time of physiological saline can be adjusted to a preferable range, and by adjusting, for example, the apparent density of the crosslinked polymer particles (A) and the weight average particle diameter of the crosslinked polymer particles to the aforementioned preferable ranges, the absorption time can be adjusted to a more preferable range.

The absorption time of physiological saline is a time measured by the following method in a room at $25 \pm 2$°C and a humidity of $50 \pm 10$%. The physiological saline is used with the temperature thereof having been adjusted beforehand to $25$°C $\pm 2$°C.

<Measurement of physiological saline absorption time>

A 100-ml beaker is charged with 1.00 g of a measurement sample, and 40 g of physiological saline (salt concentration: 0.9% by weight) is added. The mixture is left to stand without stirring, and the time taken by the complete absorption of the physiological saline (at the tail end of absorption, the beaker is inclined slightly to check whether the physiological saline remains or not) is measured and the time is taken as an absorption time (t1). The temperature of the physiological saline used and that of the measurement atmosphere are adjusted to $25$°C $\pm 2$°C.

The crosslinked polymer particles (A) are preferably crosslinked polymer particles having a basic flowability energy, as measured by a powder flow analyzer, of 500 to 8000 mJ, more preferably 1000 to 6000 mJ, and most preferably 1500 to 4000 mJ. Within such ranges, the shape retention of an absorbent article after swelling is further improved. By adjusting the contents of the hydrophobic substance (a4) and the inorganic powder (a5) and the average particle diameter and the apparent density of the crosslinked polymer particles (A) to the aforementioned preferable ranges, the basic flowability energy can be adjusted to a preferable range.

The basic flowability energy of the crosslinked polymer particles (A) is measured in accordance with the disclosure of JP-A-2007-040770 (Best Mode for Carrying Out the Inventing) and can be measured in a basic flowability energy measurement mode of a powder rheometer FT4 manufactured by Sysmex Corporation (measurement atmosphere: -25°C, 50 %RH, the amount of sample: 160 ml measured by charging a sample by free fall into a 160-ml split container having an inner diameter of 50 mm, blade width: 48 mm, rotation speed: 100 m/s, the arithmetic average of seven measurements).

From the viewpoint of the skin irritation resistance of an absorbent article, the water capacity (g/g) of the crosslinked polymer particles (A) is preferably 25 to 60, more preferably 26 to 55, and particularly preferably 27 to 50. The water retention capacity of crosslinked polymer particles is measured by the following method.

<Method of measuring water retention capacity of crosslinked polymer particles (A)>

One gram of a measurement sample is put into a tea bag (20 cm long, 10 cm wide) made of nylon net with an opening size of 63 $\mu$m (JIS Z8801-1:2006) and then is immersed in 1,000 ml of physiological saline (salt concentration: 0.9% by weight) for 1 hour without stirring, followed by draining off physiological saline by hanging the sample for 15 minutes. Then, the sample in the tea bag is put in a centrifuge and centrifugally dewatered at 150 G for 90 seconds, thereby removing excess physiological saline. Subsequently, the weight (h1) of the sample including the tea bag is measured and then a water retention capacity is calculated from the following formula.

$$\texttt{Water retention capacity (g/g) = (h1) - (h2)}$$

The temperature of the physiological saline used and that of the measurement atmosphere are adjusted to 25°C $\pm$ 2°C.

In the same way as described above except for using no measurement sample, the weight of a tea bag after centrifugal dewatering is measured, and it is expressed by (h2).

The gel elastic modulus $(N/m^2)$ of the 30-fold swollen gel obtained by allowing 30 parts by weight of an artificial urine to be absorbed into 1 part by weight of the crosslinked polymer particles (A) is preferably 2,000 to 3,000, more preferably 2,025 to 2,950, particularly preferably 2, 050 to 2, 900, and most preferably 2,075 to 2,850. Within such ranges, further improved leakage resistance is exhibited on application of the absorbent resin particles of the present invention to an absorbent article. The gel elastic modulus $(N/m^2)$ is a value determined by the following measurement method.

<Method of measuring gel elastic modulus>

In a 100 ml-volume beaker (inner diameter: 5 cm), 60.0 g of artificial urine [200 parts by weight of urea, 80 parts by weight of sodium chloride, 8 parts by weight of magnesium sulfate (7 hydrate), 3 parts by weight of calcium chloride (dihydrate), 2 parts by weight of ferric sulfate (7 hydrate), 9,704 parts by weight of ion-exchange water] was weighed, and in the same manner as the operation described in JIS K7224-1996, 2.0 g of the measurement sample is precisely weighed and charged into the beaker to prepare a 30-fold swollen gel.

The beaker containing the 30-fold swollen gel was wrapped with plastic wrap so that the swollen gel may not dry, and then the beaker is allowed to stand in an atmosphere at 40 $\pm$ 2°C for 3 hours and further in an atmosphere at 25 $\pm$ 2°C for 0.5 hours. Then, the beaker is unwrapped and the gel elastic modulus of the 30-fold swollen gel is measured using a curd meter (for example, Curd-Meter MAX ME-500 manufactured by Itec Techno Engineering K.K.). The conditions for measurement with the curd meter are as follows:

- Pressure-sensitive shaft: 8 mm
- Spring: for 100 g
- Load: 100 g
- Elevation rate: 1 inch/7 seconds
- Test property: breakage
- Measurement time: 6 seconds

- Atmospheric temperature for measurement: 25 $\pm$ 2°C

In the absorbent article of the present invention, the aqueous-liquid absorbing part includes crosslinked polymer particles (A), and may further include hydrophilic fibers (c). When the aqueous-liquid absorbing part includes crosslinked polymer particles (A) and hydrophilic fibers (c), the crosslinked polymer particles (A) and the hydrophilic fibers (c) may uniformly be mixed or alternatively may be in a form in which one of them are unevenly distributed. When the aqueous-liquid absorbing part includes the hydrophilic fibers (c), the weight ratio of the crosslinked polymer particles (A) is preferably 30% by weight or more, more preferably 50% by weight or more, particularly preferably 70% by weight or more, and most preferably 90% by weight or more, based on the total weight of the crosslinked polymer particles (A) and the hydrophilic fibers (c).

The hydrophilic fiber (c) is a hydrophilic fiber that is formed of a material which itself does not have a property to absorb an aqueous liquid and swell, and examples thereof include fibers derived from natural products having many hydroxyl groups, such as cotton-like pulp and cellulose.

The nonwoven fabric (B) contains, as an essential constituent, porous fibers (b1) as defined in the claims.

The weight ratio of the porous fibers (b1) in the nonwoven fabric (B) is preferably 20% or more and more preferably 50% or more, based on the weight of the whole nonwoven fabric (B).

The nonwoven fabric (B) can be obtained by manufacturing a nonwoven fabric by a known method using the porous fibers (b1) as a raw material and, from the viewpoint of the strength, etc. of the nonwoven fabric (B), it preferably contains non-porous fibers made of a thermoplastic resin and the porous fibers (b1) as constituents. Examples of the non-porous fiber made of a thermoplastic resin include non-porous fibers produced using known heat-fusible resins, and, for example, non-porous fibers spun by known methods using polyester, polyamide, polyethylene, polypropylene, mixtures thereof, etc. can be used.

The porous fiber (b1) is a fiber having minute pores being open towards the outside, wherein the average pore diameter of the pores of the fiber is 1000 nm or less expressed by a measurement of pore size distribution measured by the mercury press-injection method. In a reference embodiment, the porous fiber (b1) is not particularly restricted with respect to the material thereof and such polymers as polypropylene, polyester, polyacrylonitrile, and polyamide can be used.
In the invention, it is a porous fiber made of an acrylonitrile-based polymer from the viewpoint of the diffusibility of a liquid, etc. Especially, an acrylonitrile-based polymer which is produced by polymerizing a monomer composition containing acrylonitrile and has an acrylonitrile content of 70% or more based on the total weight of the monomer composition (this is also merely called a "polymer having an acrylonitrile content of 70% or more based on the total weight of the monomer composition") is preferable, an acrylonitrile-based polymer having an acrylonitrile content of 80% or more based on the total weight of the monomer composition is more preferable, and an acrylonitrile-based polymer having an acrylonitrile content of 88% by weight or more based on the total weight of the monomer composition is particularly preferable.

The acrylonitrile-based polymer to be used for the porous fibers (b1) can be obtained by homopolymerizing acrylonitrile or copolymerizing acrylonitrile with an unsaturated vinyl compound copolymerizable with acrylonitrile using well-known polymerization means such as suspension polymerization, emulsion polymerization, and solution polymerization. Examples of the unsaturated vinyl compound include the same as those enumerated as the aforementioned water-soluble vinyl monomer (a1), the aforementioned vinyl monomer (a2), and other vinyl monomers (a') copolymerizable with the foregoing.

The porous fibers (b1) can be obtained by such a known method as a method in which pores are generated by mixing a low boiling point compound or a water-absorbent resin with a fiber-forming polymer such as the aforementioned polymer and then performing a heat treatment (the methods disclosed in JP-A-61-10248 and JP-A-2000-290832) and a method in which a fiber in which a fiber-forming polymer such as the aforementioned polymer is mixed with a component having a solubility in a solvent different from the solubility of the polymer is obtained and then a solvent treatment is performed to remove a solvent-soluble component (the methods disclosed in JP-A-7-042023, JP-A-7-042017, and JP-A-6-280159).

Especially, when, according to the invention, an acrylonitrile-based polymer is used as the aforementioned polymer, preferred is a method in which from a fiber obtained by mix-spinning a polymer such as an acrylonitrile-based polymer with a water-soluble polymer or an alkali-soluble component (e.g., an acrylonitrile-based copolymer having a hydrophilic structure) is removed the water-soluble polymer or the alkali-soluble component (dissolution method), and a method in which voids are generated when an acrylonitrile-based polymer is spun (foaming method). Methods further preferable from the viewpoint of fiber strength, etc., include a dissolution method of using an acrylonitrile-based copolymer, and the porous fibers (b1) can be obtained by a method in which a soluble acrylonitrile-based polymer is removed from a fiber obtained by mix-spinning a soluble acrylonitrile copolymer having a hydrophilic structure (e.g., a polyalkylene oxide chain, a polyetheramide chain, and a polyether ester chain) formed by copolymerizing a vinyl monomer having a hydrophilic structure with a slightly soluble acrylonitrile-based polymer having 80% by weight or more of a structure derived from an acrylonitrile monomer and having no hydrophilic structure.

As the method of spinning a fiber-forming polymer such as the aforementioned polymer, there can be employed such a known method as a method in which a fiber-forming polymer such as the aforementioned polymer in a state where the polymer is molten by heat is extruded from a spinneret into a fibrous form and then is cooled to solidify the polymer in the fibrous form (melt-spinning method), a method in which a fiber-forming polymer such as the aforementioned polymer in a state where the polymer is dissolved in a solvent with low volatility is extruded into a solidification bath from a spinneret to form a fibrous form (wet-spinning method), and a method in which a fiber-forming polymer such as the aforementioned polymer in a state where the polymer is dissolved in a solvent vaporizable by heat (e.g., a low boiling point solvent) is extruded into a hot atmosphere from a spinneret, thereby allowing the solvent to vaporize and forming the polymer into a fibrous form (dry-spinning method).

The average pore diameter of the porous fibers (b1) can be adjusted, for example, in the dissolution method of using an acrylonitrile-based copolymer, by, for example, varying the ratio of the acrylonitrile-based copolymer having a hydrophilic structure to the slightly soluble acrylonitrile-based copolymer having no hydrophilic structure and the solution concentration applied during wet-spinning. The average pore diameter of the porous fibers (b1) is preferably 1 to 1000 nm, more preferably 3 to 1000 nm, even more preferably 5 to 500 nm, and particularly preferably 7 to 100 nm. It is desirable that the average pore diameter be within such ranges because both the absorption velocity and the absorption capacity for an aqueous liquid are further improved. The average pore diameter is measured by a mercury press-injection method or a gas chromatography method, and the measurement can be performed using a publicly known measuring instrument, such as "AutoPore IV 9500 series" manufactured by Shimadzu Corporation and "POREMASTER 60" manufactured by Quantachrome Corporation for the mercury penetration method, and "ASAP2020" manufactured by Shimadzu Corporation.

As the porous fibers (b1), a porous fiber having a contact angle with water with respect to the porous fiber of 80 degrees or less is preferable, and the contact angle is more preferably 70 degrees or less and particularly preferably 60 degrees or less. Although the contact angle is determined by the material of the fiber and surface treatment, a contact angle lower than 95 degrees, which is a contact angle of common polypropylene, and 75 degrees, which is a contact angle of polyester, (for example, nearly 50 degrees) can be obtained by choosing the acrylonitrile-based polymer as a fiber material. The contact angle of fiber is measured with Sigma701 manufactured by Biolin Scientific using pure water.

Although the percentage elongation of the porous fibers (b1) is adjusted by the copolymerization composition of the polymer constituting the fibers, spinning conditions, fineness, the pore diameter of a porous material, and the number of pores of the fibers, from the viewpoints of the process capability at the time of spinning and nonwoven fabric processing, the process capability at the time of processing of a product after the nonwoven fabric processing, the softness of a product, etc. , the percentage elongation is preferably 10% or more, more preferably 20% or more, and even more preferably 30% or more.

The fineness of the porous fibers (b1) is adjusted by choosing the diameter of a spinning die or the take-up speed and is preferably 0.05 to 20 dtex. The fineness is more preferably 0.1 to 10 dtex, and even more preferably 0.5 to 5 dtex. The lower the fineness, the larger the effective surface area of the fiber and the more improved the softness of a product, whereas the higher the fineness, the higher the processing capacity in a spinning step, a processing treatment step, etc. The fineness is preferably within such ranges because it becomes easy to attain the softness of a product and the processing capacity at the same time.

In order to increase the effective surface area or increase conduits for an aqueous liquid to be absorbed between fibers or in fibers, the cross-sectional shape of the fibers can appropriately be adjusted, and any of a circular shape, a triangular shape, an L shape, a hollow shape, a flat shape, etc. is available.

The strength of the porous fibers (b1) is adjusted by the copolymerization composition of the polymer constituting the fibers, the fineness, the pore diameter of a porous material, and the number of pores of the fibers, and from the viewpoints of the processing capacity at the time of spinning and/or nonwoven fabric processing, the processing capacity at the time of processing a product after the nonwoven fabric processing, the tensile strength of a product, etc., the strength is preferably 1.0 cN/dtex or more and more preferably 2.0 cN/dtex or more.

The nonwoven fabric (B) can be produced using porous fibers (b1) by a conventional method such as a needle punch method, a spun lace method, a spun bond method, a melt blow method, a chemical bond method, a thermal bond method, and a stitch bond method. The method can be chosen by taking into account the mechanical characteristics, the chemical characteristics, and the allowable cost required for a final product and the applicability of fiber materials. For example, it can be produced by a method in which a card web prepared using polyester fibers containing 20% or more porous fibers (b1) is processed into a nonwoven fabric by a needle punch method; a method in which the card web is processed into a nonwoven fabric by a chemical bond method of applying a heat-adhesive resin in an aqueous solution state to the card web and then heating and drying it; a method in which a web prepared by mixing the card web with heat-fusible binder fibers in an amount of 10 to 40% relative to the card web is processed into a nonwoven fabric by a thermal bond method of treating the web with a hot roller under a hot wind; and a method in which the card web is processed into a nonwoven fabric by a spun lace method of treating the card web by dispersing it in a high pressure jet water stream.

The nonwoven fabric (B) can further contain fibers (b2) having aqueous liquid absorbency (hereinafter referred to as aqueous-liquid absorbent fibers (b2)). By containing the aqueous-liquid absorbent fibers (b2), the nonwoven fabric (B) can absorb a liquid that has not been absorbed by aqueous-liquid absorbing parts and moreover, by distributing the aqueous-liquid absorbing parts uniformly in cavities, etc. in the nonwoven fabric (B), inefficient use of the nonwoven fabric (B) due to localized absorption and water conduction can be avoided.

The aqueous-liquid absorbent fibers (b2) are fibers that are formed of a material having a property to absorb an aqueous liquid and swell itself and examples thereof include super absorbent acrylic fibers that have a two-layer structure composed of an outer layer subjected to a super absorbing treatment and an inner layer made of acrylic fibers (the aqueous-liquid absorbent fibers disclosed in JP-B-07-061370, etc.) and super absorbent acrylic fibers that are highly absorbent fibers based on acrylic acid, methacrylic acid and an acrylic acid/methacrylic acid monomer in which the acrylic acid is partially neutralized to sodium acrylate and that are obtained by crosslinking between the polymer chains by means of ester groups made from the reaction between acid groups of the acrylic acid and hydroxyl groups in the acrylic acid/methacrylic acid monomer (the aqueous-liquid absorbent fibers disclosed in JP-W-2011-526220, etc.). The aqueous-liquid absorbent fibers (b2) are available in the market as "LANSEAL F (manufactured by Toyobo Co., Ltd.)," "OASIS SAF (manufactured by Technical Absorbents Ltd.)," etc.

When the nonwoven fabric (B) contains aqueous-liquid absorbent fibers (b2), the content of the aqueous-liquid absorbent fibers (b2) is preferably 50% or less, more preferably 30% or less, and even more preferably 10% or less, based on the total weight of the porous fibers (b1) and the aqueous-liquid absorbent fibers (b2). Examples of the method of adding the aqueous-liquid absorbent fibers (b2) to the nonwoven fabric (B) include a method in which they are mixed in a prescribed ratio prior to the preparation of a card web and a method in which a thin nonwoven fabric prepared beforehand from only the aqueous-liquid absorbent fibers (b2) is provided with other nonwoven fabric components. Since it is easy to disperse the aqueous-liquid absorbent fibers (b2) in the nonwoven fabric (B) uniformly, it is preferable to mix them in a prescribed ratio before preparing a card web.

Examples of a concrete method of obtaining the nonwoven fabric (B) containing the aqueous-liquid absorbent fibers (b2) include a method in which a card web prepared beforehand from polyester fibers prepared by mixing 60% by weight of porous fibers (b1) with 20% by weight of aqueous-liquid absorbent fibers (b2) is processed into a nonwoven fabric by a needle punch method, and a thermal bond method in which the card web and heat-fusible binder fibers in an amount of 10 to 40% relative to the card web are mixed and then subjected to a heat treatment with a hot roller, a how air, and the like.

A preferable structure of the absorbent article of the present invention can be a structure having aqueous-liquid absorbing parts having crosslinked polymer particles (A) at least on one side of the nonwoven fabric (B) and/or in cavities in the nonwoven fabric (B). Especially, the absorbent article of the present invention more preferably has a structure having aqueous-liquid absorbing parts at least on one side of the nonwoven fabric (B), and particularly preferably has a structure having aqueous-liquid absorbing parts at least on one side of the woven fabric (B) and in cavities in the nonwoven fabric (B).

The structure having aqueous-liquid absorbing parts at least on one side of a nonwoven fabric (B) and in cavities in the nonwoven fabric (B) can be obtained by, for example, a method in which in cavities of the nonwoven fabric (B) on the surface of which crosslinked polymer particles (A) are scattered uniformly are further inserted and fixed crosslinked polymer particles (A), a method in which a nonwoven fabric (B) containing heat-fusible binder fibers is heated and immediately thereafter crosslinked polymer particles (A) are scattered and fixed with the heat-fusible binder fibers, a method in which crosslinked polymer particles (A) are scattered on a nonwoven fabric (B) containing aqueous-liquid absorbent fibers (b2) and having absorbed water and then the crosslinked polymer particles (A) are fixed via water along a structure containing the aqueous-liquid absorbent fibers (b2), and a method in which a nonwoven fabric (B) is immersed in an aqueous alcohol (methanol, etc.) dispersion liquid containing crosslinked polymer particles (A) and an acrylate-based latex dispersed therein and then the crosslinked polymer particles (A) are attached to the whole nonwoven fabric (B).

The structure having aqueous-liquid absorbing parts at least on one side of a nonwoven fabric (B) can be obtained by, for example, a method in which molded crosslinked polymer (A) is laminated onto a nonwoven fabric (B), and a method in which an aqueous alcohol dispersion liquid containing crosslinked polymer particles (A) and an acrylate-based latex dispersed therein is sprayed to the surface of a nonwoven fabric (B) and then dried.

When an aqueous alcohol dispersion liquid containing crosslinked polymer particles (A) and an acrylate-based latex dispersed therein is used in the preparation of an absorbent article, the weight ratio of the crosslinked polymer particles (A) contained in the aqueous alcohol dispersion liquid is usually 10 to 80% by weight based on the total weight of the aqueous alcohol dispersion liquid and the weight ratio of crosslinked polymer particles (A) to the solid content in the latex is preferably within the range of 100/1 to 100/200. The solid content concentration of the aqueous alcohol dispersion liquid can be adjusted according to the methods of application and immersion and the required weight per unit area.

From the viewpoint that the crosslinked polymer particles (A) contained in the aqueous-liquid absorbing parts become unlikely to leave from the nonwoven fabric (B), the weight per unit area of the nonwoven fabric (B) is preferably 1 to 50 g/m$^2$, more preferably 5 to 45 g/m$^2$, and even more preferably 10 to 40 g/m$^2$.

When the absorbent article of the present invention has a structure having aqueous-liquid absorbing parts at least on one side of the nonwoven fabric (B), the absorbent article preferably has a water-permeable sheet between the nonwoven fabric (B) and the aqueous-liquid absorbing parts. As the water-permeable sheet, an air-through nonwoven fabric, a resin bond nonwoven fabric, an air-laid nonwoven fabric, a spun lace nonwoven fabric, a heat roll nonwoven fabric, a spun bond nonwoven fabric, tissue paper, a creped paper, etc. can be used. Desirably, the weight per unit area of the water-permeable sheet is 5 to 45 g/m$^2$.

The absorbent article of the present invention is allowed to have a structure having aqueous-liquid absorbing parts, a nonwoven fabric (B) and, if necessary, a water-permeable sheet on a back sheet. As the back sheet, a sheet material having water barrier properties, such as a polyethylene sheet and a urethane sheet, can be used.

The absorbent article can be obtained by publicly known production methods (JP-A-2013-255565, JP-A-2014-233447, JP-A-2003-225565, JP-A-2006-131767, JP-A-2005-097569, etc.).

EXAMPLES

The present invention is further described by examples below, but the invention is not restricted thereto. Hereafter, unless otherwise stated, "%" means "% by weight" and "part" means "part by weight."

<Production examples of crosslinked polymer particles>

<Production Example 1>

155 parts (2.15 molar parts) of a water-soluble vinyl monomer (a1) (acrylic acid), 0.6225 parts (0.0024 molar parts) of a crosslinking agent (a3) (pentaerythritol triallyl ether), and 340.27 parts of deionized water were kept at 3°C under stirring and mixing. After adjusting the dissolved oxygen amount to 1 ppm or less by introducing nitrogen into this mixture, 0.62 parts of a 1% aqueous solution of hydrogen peroxide, 1.1625 parts of a 2% aqueous solution of ascorbic acid, and 2.325 parts of a 2% aqueous solution of 2,2'-azobis[2-methyl-N-(2-hydroxyethyl)-propionamide] were added and mixed, so that polymerization was initiated. After the temperature of the mixture reached 90°C, polymerization was performed at 90 ± 2°C for about 5 hours, thereby obtaining a hydrous gel (1).

Then, while 502.27 parts of the hydrous gel (1) was chopped with a mincing machine, 128.42 parts of a 48.5% aqueous solution of sodium hydroxide was added and mixed, and subsequently 1.9 parts of a hydrophobic substance (a4) (Mg stearate) was added and mixed, so that a chopped gel (2) was obtained. Moreover, the chopped gel (2) was dried in a through-flow band type drier (at 150°C, wind speed: 2 m/second), so that a dried material was obtained. The dried material was pulverized with a juicing blender and then was adjusted to have particle sizes of 150 to 710 μm by using sieves having sizes of opening of 150, 300, 500, 600, and 710 μm, respectively, so that particles of the dried material were obtained. Under stirring 100 parts of the dried material particles at a high speed, 5 parts of a 2% water/methanol mixed solution of ethylene glycol diglycidyl ether (the weight ratio of water/methanol = 70/30) was added and mixed by spraying, followed by leaving the mixture to stand at 150°C for 30 minutes to perform surface crosslinking. Then, 0.10 parts of silica (Aerosil 200) under stirring at a high speed was added, so that crosslinked polymer particles (A-1) were obtained. The crosslinked polymer particles (A-1) had a weight average particle diameter of 400 μm and an apparent density of 0.56 g/ml. The weight average particle diameter and the apparent density were measured by the following methods, respectively.

<Measurement of weight average particle diameter>

Standard sieves having sizes of opening of 1000 μm, 850 μm, 710 μm, 500 μm, 425 μm, 355 μm, 250 μm, 150 μm, 125 μm, 75 μm, and 45 μm, respectively, were piled one on another in order, and were combined on a bottom tray. About 50 g of the crosslinked polymer particles (A-1) was put on the top sieve and then shaken for 5 minutes by a RO-TAP sieve shaker. Then, the particles remaining on the respective sieves and the bottom tray were weighed and the weight fractions of the particles on the respective sieves were calculated with the total weight of the particles considered to be 100% by weight. The calculated values were plotted on a logarithmic probability sheet {taking the size of openings of a sieve (particle diameter) as an abscissa and the weight fraction as an ordinate} and then a line connecting the respective points was drawn. Subsequently, a particle diameter that corresponded to a weight fraction of 50% by weight was determined and this was defined as a weight average particle diameter.

<Measurement of apparent density>

Measurement was performed under an environment at 25°C in accordance with JIS K7365:1999.

<Production Example 2>

Crosslinked polymer particles (A-2) were obtained in the same manner as in Production Example 1 except changing "be adjusted to have particle sizes of 150 to 710 μm by using sieves having sizes of opening of 150, 300, 500, 600, and 710 μm, respectively" to "be adjusted to have particle sizes of 150 to 600 μm by using sieves having sizes of opening of 150, 300, 500, and 600 μm, respectively." The weight average particle diameter and the apparent density of the crosslinked polymer particles (A-2) measured in the same manner as in Production Example 1 were 350 μm and 0.60 g/ml, respectively.

<Production Example 3>

Crosslinked polymer particles (A-3) were obtained in the same manner as in Production Example 1 except changing "be adjusted to have particle sizes of 150 to 710 μm by using sieves having sizes of opening of 150, 300, 500, 600, and 710 μm, respectively" to "be adjusted to have particle sizes of 150 to 500 μm by using sieves having sizes of opening of 150, 300, and 500 μm, respectively." The weight average particle diameter and the apparent density of the crosslinked polymer particles (A-3) measured in the same manner as in Production Example 1 were 300 μm and 0.64 g/ml, respectively.

<Production Example 4>

Crosslinked polymer particles (A-4) were obtained in the same manner as in Production Example 1 except changing "0.1 parts of silica (Aerosil 200)" to "0.5 parts of silica (Aerosil 200)." The weight average particle diameter and the apparent density of the crosslinked polymer particles (A-4) measured in the same manner as in Production Example 1 were 400 μm and 0.54 g/ml, respectively.

<Production Example 5>

Crosslinked polymer particles (A-5) were obtained in the same manner as in Production Example 1 except not "adding 1.9 parts of a hydrophobic substance (a4) (Mg stearate)" and not using "0.1 parts of silica (Aerosil 200) ." The weight average particle diameter and the apparent density of the crosslinked polymer particles (A-5) measured in the same manner as in Production Example 1 were 400 μm and 0.64 g/ml, respectively.

For the crosslinked polymer particles (A-1) through (A-5) obtained in Production Examples 1 through 5, the time taken to absorb physiological saline 40 times their own weight [physiological saline (40 times) absorption time], the basic flowability energy, the water retention capacity, and the gel elastic modulus were measured by the following methods, and they were shown in Table 1 together with the weight average particle diameter and the apparent density.

[Table 1]

| Production Example | | 1 | 2 | 3 | 4 | 5 |
|---|---|---|---|---|---|---|
| Crosslinked polymer particles | | A-1 | A-2 | A-3 | A-4 | A-5 |
| Physiological saline (40 times) absorption time | Second | 70 | 55 | 40 | 65 | 150 |
| Basic flowability energy | mJ | 1000 | 1500 | 2000 | 8000 | 500 |
| Weight average particle diameter | μm | 400 | 350 | 300 | 400 | 400 |
| Apparent density | g/ml | 0.56 | 0.6 | 0.64 | 0.54 | 0.64 |
| Water retention capacity | g/g | 37 | 35 | 33 | 37 | 36 |
| Gel elastic modulus | N/m$^2$ | 2600 | 2400 | 2075 | 2850 | 2500 |

<Measurement of physiological saline (40 times) absorption time>

To each of 100-ml beakers containing 1.00 g of the crosslinked polymer particles (A-1) through (A-5) obtained in Production Examples 1 through 5, respectively, was added 40 g of physiological saline (salt concentration = 0.9% by weight) . Then, they were left to stand without stirring, and the time taken by the complete absorption of the physiological saline (at the tail end of absorption, the beaker was inclined slightly to check whether the physiological saline remained or not) was measured and the time was taken as a physiological saline (40 times) absorption time. The temperature of the physiological saline used and that of the measurement atmosphere were adjusted to 25°C ± 2°C.

<Measurement of basic flowability energy>

Using a Powder Rheometer FT4 manufactured by Sysmex Corporation and set to a basic flowability energy measuring mode, measurement was repeated seven times under a measurement environment of -25°C and 50 %RH while the blade width and the rotation speed were set at 48 mm and 100 m/s, respectively, and the arithmetic average of the measurements was taken as a basic flowability energy. The measurement samples used were fixed to have a volume of 160 ml and were obtained by naturally dropping the crosslinked polymer particles (A-1) through (A-5) obtained in Production Examples 1 through 5 individually into 160-ml split containers having an inner diameter of 50 mm.

<Measurement of water retention capacity>

The crosslinked polymer particles (A-1) through (A-5) obtained in Production Examples 1 through 5 (1.00 g) were each put in a tea bag (20 cm long, 10 cm wide) formed of nylon net with an opening size of 63 $\mu$m (JIS Z8801-1:2006) and immersed in 1,000 ml of physiological saline (salt concentration: 0.9% by weight) for 1 hour without stirring. Then, the sample was lifted out of the physiological saline, followed by draining off physiological saline by hanging the sample for 15 minutes, and the sample with the tea bag was put in a centrifuge and was centrifugally dewatered at 150 G for 90 seconds to remove excessive physiological saline. The weight (h1) including the weight of the tea bag after the dewatering was measured. Moreover, the weight (h2) of a tea bag that was obtained by performing the same operation as above except not putting crosslinked polymer particles in the tea bag was measured, and then the water retention capacity was calculated from the following formula.

$$\texttt{Water retention capacity (g/g) = (h1) - (h2)}$$

The temperature of the physiological saline used and that of the measurement atmosphere were adjusted to 25°C ± 2°C.

<Measurement of gel elastic modulus>

In a 100 ml-volume beaker (inner diameter: 5 cm), 60.0 g of artificial urine [200 parts by weight of urea, 80 parts by weight of sodium chloride, 8 parts by weight of magnesium sulfate (7 hydrate), 3 parts by weight of calcium chloride (dihydrate), 2 parts by weight of ferric sulfate (7 hydrate), 9,704 parts by weight of ion-exchange water] was weighed, and in the same manner as in the operation described in JIS K7224-1996, 2.0 g of each of the crosslinked polymer particles (A-1) through (A-5) obtained in Production Examples 1 through 5 was precisely weighed and charged into the beaker to prepare a 30-fold swollen gel. Subsequently, the beaker containing the 30-fold swollen gel was wrapped with wrapping film, and then was allowed to stand in an atmosphere at 40 ± 2°C for 3 hours and further in an atmosphere at 25 ± 2°C for 0.5 hours. Then, the gel elastic modulus of the 30-fold swollen gel was measured under the following conditions using a Curd-Meter MAX ME-500 manufactured by Itec Techno Engineering K.K.

(Conditions of card meter)

- Pressure-sensitive shaft: 8 mm
- Spring: for 100 g
- Load: 100 g
- Elevation rate: 1 inch/7 seconds
- Test property: breakage
- Measurement time: 6 seconds
- Atmospheric temperature for measurement: 25 ± 2°C

<Production example of nonwoven fabric>

<Production Example 6>

A monomer mixture of acrylonitrile (90 parts), methyl acrylate (9.7 parts), and sodium methallylsulfonate (0.3 parts) was subjected to aqueous suspension polymerization, and a polymer A was thereby obtained. A monomer mixture of acrylonitrile (28 parts) and methoxypolyethylene glycol (30 mol) methacrylate (72 parts) was subjected to aqueous suspension polymerization, and a polymer B was thereby obtained. The polymer A (97 parts) and the polymer B (3 parts) were dissolved in a 50% aqueous solution of sodium rhodanate (900 parts) to form a spinning solution, and then spinning was performed using this solution to obtain porous fibers (b1-1). The fineness, the strength, the percentage elongation, the contact angle, and the average pore diameter of the resulting porous fibers (b1-1) were found to be 2 dtex, 3 cN/dtex, 50%, 46 degrees, and 10 nm, respectively. Using the porous fibers (b1-1), a nonwoven fabric (B-1) having a weight per unit area of 40 g/m$^2$ was produced by a needle punch method.

The fineness, the strength, the percentage elongation, the contact angle, and the average pore diameter of the porous fibers (b1-1) were measured by the following methods.

<Fineness>

The fineness was measured in accordance with Method A provided for in "8.5 Fineness" of JIS L1015 (2010).

<Strength and percentage elongation>

The strength and the percentage elongation were measured in accordance with the standard tests provided for in "8.7 Tensile Strength and Elongation" of JIS L1015 (2010).

<Contact angle>

One fiber was sampled out of a fiber bundle and then was immersed in and lifted from pure water using pure water as a solvent. Then, a contact angle was measured with Sigma701 manufactured by Biolin Scientific AB.

<Average Pore Diameter>

The average pore diameter was measured by the mercury press-injection method of JIS R1655 (2003).

<Production Example 7>

Porous fibers (b1-2) were obtained in the same manner as in Example 5 except using a spinning solution prepared by dissolving the polymer A (95 parts) and the polymer B (5 parts) in 900 parts of a 50% aqueous solution of sodium rhodanate. The fineness, the strength, the percentage elongation, the contact angle, and the average pore diameter of the resulting porous fibers (b1-2) were found to be 1 dtex, 2 cN/dtex, 30%, 46 degrees, and 20 nm, respectively. Using the porous fibers (b1-2), a nonwoven fabric (B-2) having a weight per unit area of 40 g/m$^2$ was produced by a needle punch method.

<Production Example 8>

A monomer mixture of acrylonitrile (75 parts) and methyl acrylate (25 parts) was subjected to aqueous suspension polymerization, and a polymer C was thereby obtained. The polymer C (97 parts) and polymer B (3 parts) were dissolved in 900 parts of a 50% aqueous solution of sodium rhodanate to form a spinning solution, and then spinning was performed using this solution to obtain porous fibers (b1-3). The fineness, the strength, the percentage elongation, the contact angle, and the average pore diameter of the resulting porous fibers (b1-3) were found to be 6 dtex, 5 cN/dtex, 70%, 50 degrees, and 40 nm, respectively. The porous fibers (b1-3) (80 parts) and polyester-based heat-fusible fibers (MELTY #4080 manufactured by UNITIKA LTD.) (20 parts) were mixed, and then a nonwoven fabric (B-3) having a weight per unit area of 40 g/m$^2$ was produced using a needle punch method and a heat press method in combination.

<Production Example 9>

A monomer mixture of acrylonitrile (94 parts), methyl acrylate (5 parts), and sodium methallylsulfonate (1 part) was subjected to solution polymerization using dimethyl sulfoxide as a polymerization solvent, and a polymer D was thereby obtained. A monomer mixture of acrylonitrile (30 parts) and methoxypolyethylene glycol (30 mol) methacrylate (70 parts) was subjected to solution polymerization, and a polymer E was thereby obtained. The polymer D (85 parts) and the polymer E (15 parts) were dissolved in dimethyl sulfoxide to obtain a spinning solution having a solid content of 15%, and then spinning was performed in a mixed solution having a dimethyl sulfoxide/water weight ratio of 1/1. Moreover, the resulting fibers were immersed in a 2% aqueous solution of sodium hydroxide to dissolve the polymer E, and porous fibers (b1-4) were thereby obtained. The fineness, the strength, the percentage elongation, the contact angle, and the average pore diameter of the porous fibers (b1-4) were found to be 2 dtex, 2 cN/dtex, 20%, 45 degrees, and 600 nm, respectively. Using the porous fibers (b1-4), a nonwoven fabric (B-4) having a weight per unit area of 40 g/m$^2$ was produced by a needle punch method.

<Production Example 10>

The porous fibers (b1-1) (90 parts) and "LANSEAL (registered trademark) F" (manufactured by Toyobo Co., Ltd., fineness: 5.6 dtex; fiber length: 51 mm) (10 parts) as aqueous-liquid absorbent fibers (b2-1) were mixed by airlaying, and then a nonwoven fabric (B-5) having a weight per unit area of 40 g/m$^2$ was obtained by a needle punch method.

<Production Example 11>

The porous fibers (b1-1) (70 parts) and the aqueous-liquid absorbent fibers (b2-1) (30 parts) were mixed by airlaying, and then a nonwoven fabric (B-6) having a weight per unit area of 40 g/m$^2$ was obtained by a needle punch method.

<Production Example 12>

The porous fibers (b1-1) (45 parts) and the aqueous-liquid absorbent fibers (b2-1) (45 parts) were mixed by airlaying and then further mixed with polyester-based heat-fusible fibers (MELTY #4080 manufactured by UNITIKA LTD.) (10 parts), and then a nonwoven fabric (B-7) having a weight per unit area of 40 g/m$^2$ was obtained using a needle punch method and a heat press method in combination.

<Production Example 13>

The porous fibers (b1-1) (10 parts) and the aqueous-liquid absorbent fibers (b2-1) (90 parts) were mixed by airlaying, and then a nonwoven fabric (B-8) having a weight per unit area of 40 g/m$^2$ was obtained by a needle punch method.

<Production Example 14>

A nonwoven fabric (B-9) having a weight per unit area of 20 g/m$^2$ was obtained from the porous fibers (b1-1) (100 parts) by a spun lace method.

<Example 1>

The crosslinked polymer particles (A-1) were uniformly scattered by hand to a weight per unit area of 200 g/m$^2$ on the nonwoven fabric (B-1) (weight per unit area: 40 g/m$^2$), followed by pressing at a pressure of 5 kg/cm$^2$ for 30 seconds to bury the crosslinked polymer particles (A-1) into cavities (i.e., micropores) in the nonwoven fabric (B-1), and thus an absorber (1) was obtained. The absorber (1) was cut into a rectangle (10 cm × 40 cm), and then the absorber (1) was sandwiched between water-permeable sheets (weight per unit area: 15.5 g/m$^2$; filter paper #2 manufactured by ADVANTEC) having the same size as the absorber (1), and further a polyethylene sheet (polyethylene film UB-1 manufactured by TAMAPOLY CO., LTD.) was arranged on the rear side as a back sheet and a nonwoven fabric (nonwoven fabric weight per unit area: 25 g/m$^2$; 2.2T 44-SMK manufactured by Toyobo Co., Ltd.) was arranged on the outermost side, and thus an absorbent article (1) was prepared.

<Example 2>

An absorbent article (2) was prepared in the same manner as in Example 1 except changing the "crosslinked polymer particles (A-1)" to the "crosslinked polymer particles (A-2)" and changing the "nonwoven fabric (B-1) to the "nonwoven fabric (B-2)."

<Example 3>

An absorbent article (3) was prepared in the same manner as in Example 1 except changing the "crosslinked polymer particles (A-1)" to the "crosslinked polymer particles (A-3)" and changing the "nonwoven fabric (B-1) to the "nonwoven fabric (B-3) ."

<Example 4>

An absorbent article (4) was prepared in the same manner as in Example 1 except changing the "crosslinked polymer particles (A-1)" to the "crosslinked polymer particles (A-4)" and changing the "nonwoven fabric (B-1) to the "nonwoven fabric (B-4) ."

<Example 5>

An absorbent article (5) was prepared in the same manner as in Example 1 except changing the "crosslinked polymer particles (A-1)" to the "crosslinked polymer particles (A-5)" and changing the "nonwoven fabric (B-1) to the "nonwoven fabric (B-5)."

<Example 6>

An absorbent article (6) was prepared in the same manner as in Example 1 except changing the "nonwoven fabric (B-1)" to the "nonwoven fabric (B-6)."

<Example 7>

Hydrophilic fibers (c) (fluff pulp) (20 parts) and 80 parts of the crosslinked polymer particles (A-1) were mixed using an air-flow type mixing apparatus (pad former) to obtain a mixture, and then the mixture was uniformly stacked to a weight per unit area of 250 $g/m^2$ on an acrylic plate (4 mm thick), followed by pressing at a pressure of 5 $kg/cm^2$ for 30 seconds, and thus an absorber (2) was obtained. The absorber (2) was cut into a rectangle sized in 10 cm $\times$ 40 cm, and water-permeable sheets (weight per unit area: 15.5 $g/m^2$; filter paper #2 manufactured by ADVANTEC) having the same size as the absorber were arranged on both sides of the absorber, and further a polyethylene sheet (polyethylene film UB-1 manufactured by TAMAPOLY CO., LTD.) was arranged on the rear side as a back sheet, a nonwoven fabric (B-7) (weight per unit area: 40 $g/m^2$) was arranged on the front side, and a nonwoven fabric (nonwoven fabric weight per unit area: 25 $g/m^2$; 2.2T 44-SMK manufactured by Toyobo Co., Ltd.) was arranged on the outermost side, and thus an absorbent article (7) was prepared. The weight ratio of the crosslinked polymer particles to the hydrophilic fibers (weight of crosslinked polymer particles/weight of hydrophilic fibers) was 80/20.

<Example 8>

An absorbent article (8) was prepared in the same manner as in Example 1 except changing the "nonwoven fabric (B-1)" to the "nonwoven fabric (B-8)."

<Example 9>

An absorbent article (9) was prepared in the same manner as in Example 7 except changing the amount of the hydrophilic fibers (c) from 20 parts to 5 parts and the amount of the crosslinked polymer particles (A-1) from 80 parts to 95 parts and also changing the "nonwoven fabric (B-7)" to the "nonwoven fabric (B-1)."
<Example 10>
An absorbent article (10) was prepared in the same manner as in Example 7 except changing the "nonwoven fabric (B-7)" to the "nonwoven fabric (B-1)."

<Example 11>

An absorbent article (11) was prepared in the same manner as in Example 7 except changing the amount of the hydrophilic fibers (c) from 20 parts to 80 parts and the amount of the crosslinked polymer particles (A-1) from 80 parts to 20 parts and also changing the "nonwoven fabric (B-7)" to the "nonwoven fabric (B-8)."

<Example 12>

An absorbent article (12) was prepared in the same manner as in Example 7 except changing the amount of the hydrophilic fibers (c) from 20 parts to 50 parts and the amount of the crosslinked polymer particles (A-1) from 80 parts to 50 parts.

<Example 13>

An absorbent article (13) was prepared in the same manner as in Example 7 except changing the "nonwoven fabric (B-7)" to the "nonwoven fabric (B-9)."

<Example 14>

An absorbent article (14) was prepared in the same manner as in Example 7 except changing the "nonwoven fabric (B-7)" to the "nonwoven fabric (B-3)."

<Comparative Example 1>

An absorbent article (H1) was prepared in the same manner as in Example 7 except not using the nonwoven fabric (B-7) containing the porous fibers, and instead using a nonwoven fabric (HB-1) containing no porous fibers (nonwoven fabric weight per unit area: 25 $g/m^2$, 2.2T 44-SMK manufactured by Toyobo Co., Ltd.).

<Comparative Example 2>

An absorbent article (H2) was prepared in the same manner as in Example 1 except changing the crosslinked polymer particles (A-1) to the hydrophilic fibers (c) (fluff pulp) and the "nonwoven fabric (B-1)" to the "nonwoven fabric (B-8)."

For the absorbent articles (1 through 14) obtained in Examples 1 through 14 and the comparative absorbent articles (H1 and H2) obtained in Comparative Examples 1 and 2, shape retention was evaluated by the following method and the results are shown in Table 2.

<Measurement of shape retention>

Each of the absorbent articles obtained in Examples 1 through 14 and Comparative Examples 1 and 2 was cut at the center portion into a size of 8 cm × 3 cm with scissors and the cut piece was put into a bag with a zipper (10 cm × 14 cm). After filling the bags with nitrogen gas, the bags were zipped, and each of the bags was shaken for 10 seconds by hand 10 times. The zipper was opened and the cut sample was allowed to absorb 12 g of physiological saline. After 5 minutes from the charge of the physiological saline, the bag was filled with nitrogen gas again and was zipped, and each of the bags was shaken for 20 seconds by hand 20 times. Then, the shape of the cut sample was checked and was evaluated by classifying into levels 1 to 5 based on the following criteria.

1: The sample is in a disjointed form;
2: The sample is in a form composed mostly of disjointed parts and partly of massive parts;
3: The sample is in a form composed evenly of disjointed parts and massive parts;
4: The sample is in a form composed mostly of massive parts and partly of disjointed parts; and
5: The sample is in a massive form.

[Table 2]

| No. | | Absorbent article | Aqueous-liquid absorbing part | | Liquid diffusion part | Retention of shape |
|---|---|---|---|---|---|---|
| | | | Crosslinked polymer particles (A) | Hydrophilic fibers (c) | Nonwoven fabric (B) | |
| | 1 | 1 | A-1 | No | B-1 | 5 |
| | 2 | 2 | A-2 | No | B-2 | 5 |

(continued)

| No. | | Absorbent article | Aqueous-liquid absorbing part | | Liquid diffusion part | Retention of shape |
|---|---|---|---|---|---|---|
| | | | Crosslinked polymer particles (A) | Hydrophilic fibers (c) | Nonwoven fabric (B) | |
| Example | 3 | 3 | A-3 | No | B-3 | 4 |
| | 4 | 4 | A-4 | No | B-4 | 4 |
| | 5 | 5 | A-5 | No | B-5 | 3 |
| | 6 | 6 | A-1 | No | B-6 | 4 |
| | 7 | 7 | A-1 | 20 parts | B-7 | 4 |
| | 8 | 8 | A-1 | No | B-8 | 3 |
| | 9 | 9 | A-1 | 5 parts | B-1 | 4 |
| | 10 | 10 | A-1 | 20 parts | B-1 | 5 |
| | 11 | 11 | A-1 | 80 parts | B-8 | 2 |
| | 12 | 12 | A-1 | 50 parts | B-7 | 3 |
| | 13 | 13 | A-1 | 20 parts | B-9 | 5 |
| | 14 | 14 | A-1 | 20 parts | B-3 | 4 |
| Comparative Example | 1 | H2 | No | 100 parts | B-8 | 1 |
| | 2 | H1 | A-1 | 20 parts | HB-1 | 1 |

As shown in Table 2, the absorbent articles of the present invention were superior to comparative absorbent articles in absorber retention after swelling. Therefore, it is easily expected that when the absorbent article of the present invention is used, it is superior in the retention of the shape of an absorber and in the absorbability for an aqueous liquid even when an external force is applied thereto, and even if a certain force is continuously or discontinuously applied to an absorbing site, neither rupture nor twist of the absorbing part occurs and liquid leakage is not caused by degradation of absorption performance, and rash of the skin, etc. derived therefrom are not caused.

INDUSTRIAL APPLICABILITY

The absorbent article of the present invention is useful for a disposable diaper for children, a disposable diaper for adults, a sanitary napkin, a pet sheet, a pantyliner, an incontinence pad, a sweat absorbent sheet, a blood absorbent article for medical use, a wound-protecting material, a wound-healing agent, a surgical drainage disposal agent, etc.

**Claims**

1.   An absorbent article comprising:

an aqueous-liquid absorbing part that contains crosslinked polymer particles (A) comprising, as essential constitutional units, a water-soluble vinyl monomer (a1) and/or a vinyl monomer (a2) that turns into a water-soluble vinyl monomer (a1) through hydrolysis, and a crosslinking agent (a3); and
a nonwoven fabric (B) comprising, as an essential constituent, porous fibers (b1), wherein the porous fibers (b1) are formed of an acrylonitrile-based polymer.

2.   The absorbent article according to claim 1, wherein the crosslinked polymer particles (A) are crosslinked polymer particles that absorb physiological saline 40 times their own weight in 40 to 150 seconds.

3.   The absorbent article according to claim 1 or 2, wherein the crosslinked polymer particles (A) are crosslinked polymer particles having a basic flowability energy measured by a powder flow analyzer of 500 to 8000 mJ.

4. The absorbent article according to any one of claims 1 to 3, wherein the aqueous-liquid absorbing part further contains hydrophilic fibers (c).

5. The absorbent article according to any one of claims 1 to 4, wherein the porous fibers (b1) are formed of an acrylonitrile-based polymer that is formed by polymerizing a monomer composition containing acrylonitrile and has an acrylonitrile content of 70% or more based on the total weight of the monomer composition.

6. The absorbent article according to any one of claims 1 to 5, wherein the average pore diameter of the porous fibers (b1) is 1 to 1000 nm.

7. The absorbent article according to any one of claims 1 to 6, wherein the contact angle with pure water with respect to the porous fibers (b1) is 80 degrees or less.

8. The absorbent article according to any one of claims 1 to 7, wherein the percentage elongation of the porous fibers (b1) is 10% or more.

9. The absorbent article according to any one of claims 1 to 8, wherein the fineness of the porous fibers (b1) is 0.05 to 20 dtex.

10. The absorbent article according to any one of claims 1 to 9, wherein the strength of the porous fibers (b1) is 1.0 cN/dtex or more.

11. The absorbent article according to any one of claims 1 to 10, wherein the nonwoven fabric (B) further comprises aqueous-liquid absorbent fibers (b2).

12. The absorbent article according to claim 11, wherein the content of the aqueous-liquid absorbent fibers (b2) is less than 50% based on the total weight of the porous fibers (b1) and the aqueous-liquid absorbent fibers (b2).

13. The absorbent article according to any one of claims 1 to 12, wherein the absorbent article has the aqueous-liquid absorbing part at least on one side of the nonwoven fabric (B) and/or in cavities in the nonwoven fabric (B).

14. The absorbent article according to claim 13, wherein the absorbent article has the aqueous-liquid absorbing part at least on one side of the nonwoven fabric (B) and further has a water-permeable sheet between the aqueous-liquid absorbing part and the nonwoven fabric (B).

**Patentansprüche**

1. Ein absorbierender Gegenstand, umfassend:

einen wässrige Flüssigkeit absorbierenden Teil, der vernetzte Polymerteilchen (A) enthält, welche als wesentliche konstituierende Einheiten ein wasserlösliches Vinylmomoner (a1) und/oder ein Vinylmonomer (a2), das durch Hydrolyse zu einem wasserlöslichen Vinylmonomer (a1) wird, und ein Vernetzungsmittel (a3) umfassen; und
einen Vliesstoff (B), der als eine wesentliche Komponente poröse Fasern (b1) umfasst, wobei die porösen Fasern (b1) aus einem Polymer auf Acrylnitrilbasis gebildet sind.

2. Der absorbierende Gegenstand nach Anspruch 1, wobei die vernetzten Polymerteilchen (A) vernetzte Polymerteilchen sind, die das 40-Fache ihres eigenen Gewichts an physiologischer Kochsalzlösung in 40 bis 150 Sekunden absorbieren.

3. Der absorbierende Gegenstand nach Anspruch 1 oder 2, wobei die vernetzten Polymerteilchen (A) vernetzte Polymerteilchen mit einer Basic Flowability Energy, gemessen mit einem Powder Flow Analyzer, von 500 bis 8000 mJ, sind.

4. Der absorbierende Gegenstand nach einem der Ansprüche 1 bis 3, wobei der wässrige Flüssigkeit absorbierende Teil ferner hydrophile Fasern (c) enthält.

5. Der absorbierende Gegenstand nach einem der Ansprüche 1 bis 4, wobei die porösen Fasern (b1) aus einem Polymer auf Acrylnitrilbasis gebildet sind, das durch Polymerisieren einer Monomerzusammensetzung, die Acrylnitril enthält und einen Acrylnitrilgehalt von 70% oder mehr, bezogen auf das Gesamtgewicht der Monomerzusammensetzung, aufweist, gebildet wird.

6. Der absorbierende Gegenstand nach einem der Ansprüche 1 bis 5, wobei der durchschnittliche Porendurchmesser der porösen Fasern (b1) 1 bis 1000 nm beträgt.

7. Der absorbierende Gegenstand nach einem der Ansprüche 1 bis 6, wobei der Kontaktwinkel mit reinem Wasser, bezogen auf die porösen Fasern (b1), 80 Grad oder weniger beträgt.

8. Der absorbierende Gegenstand nach einem der Ansprüche 1 bis 7, wobei die prozentuale Dehnung der porösen Fasern (b1) 10% oder mehr beträgt.

9. Der absorbierende Gegenstand nach einem der Ansprüche 1 bis 8, wobei die Feinheit der porösen Fasern (b1) 0,05 bis 20 dtex beträgt.

10. Der absorbierende Gegenstand nach einem der Ansprüche 1 bis 9, wobei die Stärke der porösen Fasern (b1) 1,0 cN/dtex oder mehr beträgt.

11. Der absorbierende Gegenstand nach einem der Ansprüche 1 bis 10, wobei der Vliesstoff (B) ferner wässrige Flüssigkeit absorbierende Fasern (b2) umfasst.

12. Der absorbierende Gegenstand nach Anspruch 11, wobei der Gehalt der wässrige Flüssigkeit absorbierenden Fasern (b2) weniger als 50% beträgt, bezogen auf das Gesamtgewicht der porösen Fasern (b1) und der wässrige Flüssigkeit absorbierenden Fasern (b2).

13. Der absorbierende Gegenstand nach einem der Ansprüche 1 bis 12, wobei der absorbierende Gegenstand den wässrige Flüssigkeit absorbierenden Teil auf mindestens einer Seite des Vliesstoffes (B) und/oder in Hohlräumen im Vliesstoff (B) aufweist.

14. Der absorbierende Gegenstand nach Anspruch 13, wobei der absorbierende Gegenstand den wässrige Flüssigkeit absorbierenden Teil auf mindestens einer Seite des Vliesstoffes (B) aufweist und ferner eine wasserdurchlässige Schicht zwischen dem wässrige Flüssigkeit absorbierenden Teil und dem Vliesstoff (B) aufweist.

**Revendications**

1. Article absorbant comprenant :

    une partie absorbant les liquides aqueux qui contient des particules polymères réticulées (A) comprenant, à titre de motifs constitutifs essentiels, un monomère de vinyle hydrosoluble (a1) et/ou un monomère de vinyle (a2) qui est converti en monomère de vinyle hydrosoluble (a1) par hydrolyse, et un agent de réticulation (a3) ; et un non-tissé (B) comprenant, à titre de constituant essentiel, des fibres poreuses (b1), dans lequel les fibres poreuses (b1) sont formées d'un polymère à base d'acrylonitrile.

2. Article absorbant selon la revendication 1, dans lequel les particules polymères réticulées (A) sont des particules polymères réticulées qui absorbent le sérum physiologique à raison de 40 fois leur poids en 40 à 150 secondes.

3. Article absorbant selon la revendication 1 ou 2, dans lequel les particules polymères réticulées (A) sont des particules polymères réticulées ayant une énergie de fluidité de base mesurée par un analyseur d'écoulement de poudre de 500 à 8000 mJ.

4. Article absorbant selon l'une quelconque des revendications 1 à 3, dans lequel la partie absorbant les liquides aqueux contient en outre des fibres hydrophiles (c).

**5.** Article absorbant selon l'une quelconque des revendications 1 à 4, dans lequel les fibres poreuses (b1) sont formées d'un polymère à base d'acrylonitrile qui est formé par polymérisation d'une composition monomère contenant de l'acrylonitrile et a une teneur en acrylonitrile de 70 % ou plus sur la base du poids total de la composition monomère.

**6.** Article absorbant selon l'une quelconque des revendications 1 à 5, dans lequel le diamètre de pore moyen des fibres poreuses (b1) est de 1 à 1000 nm.

**7.** Article absorbant selon l'une quelconque des revendications 1 à 6, dans lequel l'angle de contact avec l'eau pure par rapport aux fibres poreuses (b1) est de 80 degrés ou moins.

**8.** Article absorbant selon l'une quelconque des revendications 1 à 7, dans lequel l'allongement en pourcentage des fibres poreuses (b1) est de 10 % ou plus.

**9.** Article absorbant selon l'une quelconque des revendications 1 à 8, dans lequel la finesse des fibres poreuses (b1) est de 0,05 à 20 dtex.

**10.** Article absorbant selon l'une quelconque des revendications 1 à 9, dans lequel la force des fibres poreuses (b1) est de 1,0 cN/dtex ou plus.

**11.** Article absorbant selon l'une quelconque des revendications 1 à 10, dans lequel le non-tissé (B) comprend en outre des fibres absorbant les liquides aqueux (b2).

**12.** Article absorbant selon la revendication 11, dans lequel la teneur en fibres absorbant les liquides aqueux (b2) est inférieure à 50 % sur la base du poids total des fibres poreuses (b1) et des fibres absorbant les liquides aqueux (b2).

**13.** Article absorbant selon l'une quelconque des revendications 1 à 12, dans lequel l'article absorbant comprend la partie absorbant les liquides aqueux au moins d'un côté du non-tissé (B) et/ou dans des cavités dans le non-tissé (B).

**14.** Article absorbant selon la revendication 13, dans lequel l'article absorbant comprend la partie absorbant les liquides aqueux au moins d'un côté du non-tissé (B) et comprend en outre une feuille perméable à l'eau entre la partie absorbant les liquides aqueux et le non-tissé (B).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3916852 B
- JP 5404959 B
- US 2011301560 A1
- JP H07150470 A
- JP H05311508 A
- US 5436275 A
- JP 3648553 B
- JP 2003165883 A
- JP 2005075982 A
- JP 2005095759 A
- JP 55133413 A
- JP 54030710 B
- JP 56026909 A
- JP 1005808 A
- JP 59189103 A
- JP 58180233 A
- JP 61016903 A
- JP 61211305 A
- JP 61252212 A
- JP 51136588 A
- JP 61257235 A
- JP 2003225565 A
- JP 2006131767 A
- JP 2007040770 A
- JP 61010248 A
- JP 2000290832 A
- JP 7042023 A
- JP 7042017 A
- JP 6280159 A
- JP 7061370 B
- JP 2011526220 W
- JP 2013255565 A
- JP 2014233447 A
- JP 2005097569 A

### Non-patent literature cited in the description

- Perry's Chemical Engineers' Handbook. McGraw-Hill Book Company, 1984, 21